# EUROPEAN PATENT APPLICATION

(11) **EP 4 279 501 A2**
(43) Date of publication of application: **22.11.2023**
(21) Application number: 23177824.2
(22) Date of filing: 14.02.2019
(51) Int. Cl.: C07K 14/725

(54) **TUMOR ENVIRONMENT-SPECIFIC EXPRESSION OF CHIMERIC ANTIGEN RECEPTORS**

(30) Priority: 15.02.2018 US 201862631095 P; 23.12.2018 US 201862784501 P
(62) Divisional of application: 19713223.6
(71) Applicant: The National Institute for Biotechnology in the Negev Ltd., 8410501 Beer-Sheva (IL)
(72) Inventor: PORGADOR, Angel, 8533800 Lehavim (IL); GAZIT, Roi, 7079500 Kidron (IL)
(74) Representative: AWA Denmark A/S

(57) **Abstract**

A Tumor Micro-Environment (TME) responsive expression vector including a nucleic acid sequence encoding a synthetic promoter comprising one or more promoter-response-elements, and a nucleic acid sequence encoding immune-effector genes, such as chimeric antigen receptor. The TME responsive vector is designed to induce the expression of immune-effector genes within TME, and not in normal healthy tissues, thus focusing immune activities, increasing safety and reducing the ON-target OFF-tumor hazard.

## Description

### FIELD OF INVENTION

The present disclosure generally relates to the field of chimeric antigen receptor (CAR) expression, specifically to tumor tissue specific CAR expression.

### BACKGROUND

Harnessing the immune system to eradicate cancer has proved highly efficient in recent years.

An example is engineered immune cells such as Chimeric-Antigen-Receptor T-cells (CAR-T), which have been approved by the FDA for the treatment of various cancers after outstanding results were shown regarding the ability to eradicate malignancies that had no other efficient treatment.

However, although CAR expressing immune cells can reach tumors and metastasis throughout a patient's body, the specificity of the engineered receptor does not allow fully distinguishing between tumor cells and normal cells, with a few exceptions, since the tumor often does not have an absolutely unique antigen that is not expressed by some normal cells in the body. As a result, several CAR treatments caused toxic immune response, similar to GVHD, and even death that resulted from the CAR treatment during clinical trials.

Attempts to achieve non-constitutive expression of CAR within engineered immune cells have been made. An example includes applying an "ON-OFF switch" within the CAR expression vector, by utilizing a promoter activated only in the presence of an exogenously provided molecule (such as tetracycline/doxycycline). Albeit allowing turning off the CAR expression in case adverse symptoms is pronounced, this approach also turns off the positive activity of the CAR T-cells against the tumor cells, and thus terminates a potent CAR treatment.

There thus remains an unmet need for controlled CAR expression that reduces the risks of its life-threatening "side-effect", while allowing effective elimination of tumors.

### SUMMARY

The following embodiments and aspects thereof are described and illustrated in conjunction with compositions and methods which are meant to be exemplary and illustrative, not limiting in scope. In various embodiments, one or more of the above-described problems have been reduced or eliminated, while other embodiments are directed to other advantages or improvements.

According to some embodiments, there is provided a novel platform for regulation of effector gene expression under the control of tumor-microenvironment-responsive promoters, optionally in conjunction with a tet-response circuit.

The platform includes a tumor environment (TME) responsive expression vector including a nucleic acid sequence encoding a synthetic promoter comprising one or more TME dependent promoter response element; conjugated to effector-genes such as, but not limited to, nucleic acid sequence encoding an effector gene (e.g. CAR).

Advantageously, the TME responsive vector is designed such that binding of one or more factors, present in the TME, to the promoter response element, either directly or indirectly, induces expression by the promoter. In the absence of TME factors binding, the promoter expression is downregulated autonomously within each of the engineered cells. This advantageously ensures that minimal to no expression of effector-mechanisms, such as CAR, is found in tissue environments different from that of the tumor; whereas in the tumor environment, the expression of effector mechanisms, such as CAR, is upregulated and directing activities against the tumor while sparing normal tissues.

According to some embodiments, the expression vector may include more than one TME dependent promoter response element. This may serve to ensure that the highest expression level is solely obtained where the specific combination of TME factors is found.

Advantageously, we may optionally further replace/change the synthetic promoter for a custom-made promoter, such that the one or more TME dependent promoter response elements, configured to activate the promoter, will fit the actual TME signature of a specific patient or patient group, thus ensuring an uttermost specific and efficient response.

According to some embodiments, there is provided a tumor microenvironment (TME) responsive expression vector comprising a nucleic acid sequence encoding a synthetic promoter, said promoter comprising one or more TME dependent promoter response elements (PRE); and a nucleic acid sequence encoding an effector gene; wherein said TME responsive expression vector is designed, such that binding of one or more TME factors present in the TME to the promoter response element induces expression of the effector-gene, and wherein, in the absence of binding of the one or more TME factor to the promoter response element, low or essentially no effector gene is expressed.

According to some embodiments, the CAR is a chimeric antigen T-cell receptor (CAR-T), a chimeric antigen Natural Killer (NK) cell receptor (CAR-NK), a chimeric innate receptor, other immune-effectors including, but not limited to, cytokines, chemokines, chemokine-receptors, proteases, micro-RNAs, or combinations thereof.

According to some embodiments, the promoter response element comprises one or more response elements selected from the list consisting of, but not limited to: an interferon-gamma (IFN-γ) element response, a Nuclear Factor kappa-B (NF-κB) response element, a hypoxia response element, an IL-6 response elements, a Heat shock protein 70 (HSP-70) response element, an IL-1 response element, an IL-4 response elements, an IL-6 response elements, an IL-8 response element, an IL-10 response element, an IL-11 response element, an IL-12 response element, an IL-15 response element, an IL-18 response element, an IL-17 response element, an IL-21 response element, an IL-35 response element, a TGF-beta response element, a GM-CSF response element, a Hepatic Growth Factor (HGF) response element, an Aryl Hydrogen Receptor (AhR) response element, a PGE2 response element or any other suitable TME factor response element or combinations thereof.

According to some embodiments, the promoter response element comprises one or more response elements selected from the list consisting of, but not limited to: an interferon-gamma (IFN-γ) element response, a Nuclear Factor kappa-B (NF-κB) response element, a hypoxia response element, an IL-1 response element, an IL-6 response elements, an IL-8 response element, an IL-11 response element, an IL-12 response element, an IL-15 response element, an IL-18 response element, an IL-17 response element, an IL-21 response element, a TGF-beta response element, a GM-CSF response element, a Hepatic Growth Factor (HGF) response element, an Aryl Hydrogen Receptor (AhR) response element, or any other suitable TME factor response element or combinations thereof.

According to some embodiments, the promoter response element comprises one or more response elements selected from the list consisting of, but not limited to: an interferon-gamma (IFN-γ) element response, a Nuclear Factor kappa-B (NF-κB) response element, a hypoxia response element, an IL-6 response element, a Heat shock protein 70 (HSP-70) response element or any other suitable TME factor response element or combinations thereof.

According to some embodiments, the promoter response element may be inserted into the synthetic promoter in a sense (5' to 3') or anti-sense (3' to 5') direction.

According to some embodiments, the promoter response element comprises a nucleic acid selected from the group consisting of: TTCCGGGAA set forth in SEQ ID NO. 1, GGGAATTTCC set forth in SEQ ID NO. 2, GACCTTGAGTACGTGCGTCTCTGCACGTATG set forth in SEQ ID NO. 3, GCGCTTCCTGACAGTGACGCGAGCCG set forth in SEQ ID NO. 4, GCGCTTCCTGACAGTGACGCGAGCCG, or any combination thereof.

According to some embodiments, the promoter response element comprises a nucleic acid selected from the group consisting of: 
TACAGGGACAGCAGAGATCCAGTTTGGACTAGCCCGGTCGCACTAGTTCT
AGAACTTCCCGGAAATAGGGTGGGCAAGTATTTCCGGGAAATTCTAGAGG
GAGTTCCCGGGGACTTTCCGGGGATTTTCTCTAGATATTAAGGTGACGCGT
 
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 22, 
TACAGGGACAGCAGAGATCCAGTTTGGACTAGCCCGGTCGCACTAGTTCT
AGAACTTCCCGGAAGTAGGGTGGGCAAGTACTTCCCGGAAGTTCTAGAGG
AAATTTTCGGGGACTTTCCGGGGGTTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 23, 
TACAGGGACAGCAGAGATCCAGTTTGGACTAGCCCGGTCGCACTAGTTCT
AGAACTTCCCGGAAGTAGGGTGGGCAAGTACTTCCGGGAAATTCTAGAGG
GAGTTCTCGGGGACTTTCCGGGAATTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 24, 
TACAGGGACAGCAGAGATCCAGTTTGGACTAGCCCGGTCGCACTAGTTCT
AGAACTTCCCGGAAGTAGGGTGGGCAAGTATTTCCCGGAAGTTCTAGAGG
AAGTTCTCGGGGACTTTCCGGAGATTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 25, 
TACAGGGACAGCAGAGATCCAGTTTGGACTAGCCCGGTCGCACTAGTTCT
AGAACTTCCGGGAAATAGGGTGGGCAAGTACTTCCGGGAAATTCTAGAGG
GGATTTCCGGGGACTTTCCGGAGGTTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 26, 
TACAGGGACAGCAGAGATCCAGTTTGGACTAGCCCGGTCGCACTAGTTCT
AGAACTTCCGGGAAATAGGGTGGGCAAGTACTTCCGGGAAGTTCTAGAGG
AGGTTTTCGGGGACTTTCCGGAGGTTTCCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 27, 
TACAGGGACAGCAGAGATCCAGTTTGGACTAGCCCGGTCGCACTAGTTCT
AGAACTTCCGGGAAGTAGGGTGGGCAAGTATTTCCCGGAAGTTCTAGAGG
GGGTTTTCGGGGACTTTCCGGAGGTTTCCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 28, 
TACAGGGACAGCAGAGATCCAGTTTGGACTAGCCCGGTCGCACTAGTTCT
AGAACTTCCGGGAAGTAGGGTGGGCAAGTATTTCCGGGAAATTCTAGAGG
GAGTTCTCGGGGACTTTCCGGGGATTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 29, 
TACAGGGACAGCAGAGATCCAGTTTGGACTAGCCCGGTCGCACTAGTTCT
AGAATTTCCCGGAAATAGGGTGGGCAAGTACTTCCGGGAAATTCTAGAGG
GGGTTTCCGGGGACTTTCCGGGGGTTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 30, 
TACAGGGACAGCAGAGATCCAGTTTGGACTAGCCCGGTCGCACTAGTTCT
AGAATTTCCCGGAAATAGGGTGGGCAAGTACTTCCGGGAAATTCTAGAGG
GGGTTTCCGGGGACTTTCCGGGGGTTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 31, 
TACAGGGACAGCAGAGATCCAGTTTGGACTAGCCCGGTCGCACTAGTTCT
AGAATTTCCCGGAAATAGGGTGGGCAAGTATTTCCGGGAAATTCTAGAGG
AGGTTCTCGGGGACTTTCCGGGAGTTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 32, 
TACAGGGACAGCAGAGATCCAGTTTGGACTAGCCCGGTCGCACTAGTTCT
AGAATTTCCCGGAAATAGGGTGGGCAAGTATTTCCGGGAAATTCTAGAGG
AGGTTTTCGGGGACTTTCCGGGAGTTTCCTCTAGATATTAAGGTGACGCGT
GTGGCCTCGAACACCGAGCGACCCTGCAGCGACCCGCTTAAAAGCGGCCG
 CCATGGGCCGCCATGGCCTCCTCCGAGGACGTCATCAAGGAGTTCATGC
>set forth in SEQ ID NO: 33, 
TACAGGGACAGCAGAGATCCAGTTTGGACTAGCCCGGTCGCACTAGTTCT
AGAATTTCCCGGAAATAGGGTGGGCAAGTATTTCCGGGAAGTTCTAGAGG
AAGTTTTCGGGGACTTTCCGGGAATTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 34, 
TACAGGGACAGCAGAGATCCAGTTTGGACTAGCCCGGTCGCACTAGTTCT
AGAATTTCCGGGAAATAGGGTGGGCAAGTATTTCCCGGAAGTTCTAGAGG
AGATTCTCGGGGACTTTCCGGGGGTTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 35, 
TACAGGGACAGCAGAGATCCAGTTTGGACTAGCCCGGTCGCACTAGTTCT
AGAATTTCCGGGAAGTAGGGTGGGCAAGTACTTCCGGGAAATTCTAGAGG
GGGTTTCCGGGGACTTTCCGGAGATTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 36, 
TACAGGGACAGCAGAGATCCAGTTTGGACTAGCCCGGTCGCACTAGTTCT
AGAATTTCCGGGAAATAGGGTGGGCAAGTATTTCCCGGAAGTTCTAGAGG
GGGTTTTCGGGGACTTTCCGGAAATTTTCTCTAGATATTAAGGTGACGCGT
GTGGCCTCGAACACCGAGCGACCCTGCAGCGACCCGCTTAAAAGCGGCCG
CCATGGGCCCCATGGCCTCCTCCGAGGACGTCATCAAGGAGTTCATGC >set
forth in SEQ ID NO: 37, 
TACAGGGACAGCAGAGATCCAGTTTGGACTAGCCCGGTCGCACTAGTTCT
AGAACTTCCCGGAAATAGGGTGGGCAAGTATTTCCCGGAAGTTCTAGAGG
AGGTTTTCGGGGACTTTCCGGGATTCCCTCTAGATATTAAGGTGACGCGTG
TGGCCTCGAACACCGAGCGACCCTGCAGCGACCCGCTTAAAAGCGGCCGC
CATGGGCCGCCATGGCCTCCTCCGAGGACGTCATCAAGGAGTTCATGC >set
forth in SEQ ID NO: 38, 
TACAGGGACAGCAGAGATCCAGTTTGGACTAGCCCGGTCGCACTAGTTCT
AGTTCCGGGAAGTGGGTGGGCAATATTTCCCGGAAGTTTAGAGGAAGTTT
 
TCGGGGACTTCCGGAAATTCCCTCTAGATATTAAGGTGACGCGTGTGGCC
TCGAACACCGAGCGACCCTGCAGCGACCCGCTTAAAAGCGGCCGCCATGG
GCCGCCATGGCCTCCTCCGAGGACGTCATCAAGGAGTTCATGC >set forth in
SEQ ID NO: 39, 
TACAGGGACAGCAGAGATCCAGTTTGGACTAGCCCGGTCGCACTAGTTCT
AGAACTTCTCGGAAATAGGGTGGGCAAGTACTGTGGCCTCGAACACCGAG
CGACCCTGCAGCGACCCGCTTAAAAGCGGCCGCCATGGGCCGCCATGGCC
TCCTCCGAGGACGTCATCAAGGAGTTCATGCAGACCAAGTCTCTGCTACC
>set forth in SEQ ID NO: 40 or combinations thereof. Each possibility is a separate embodiment.

According to some embodiments, the promoter response element comprises a nucleic acid selected from the the nucleic acid sequences set forth in SEQ ID Nos 1-4 or any combination thereof. Each possibility is a separate embodiment.

According to some embodiments, the promoter response element comprises a nucleic acid selected from the nucleic acid sequences set forth in SEQ ID Nos 22-40 or any combination thereof. Each possibility is a separate embodiment.

According to some embodiments, the promoter response element comprises the nucleic acid sequence: 
TACAGGGACAGCAGAGATCCAGTTTGGACTAGCCCGGTCGCACTAGTTCT
AGAAYTTCCSGGAARTAGGGTGGGCAAGTAYTTCCSGGAARTTCTAGAGG
RRRTTYYCGGGGACTTTCCGGRRRTTYYCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
 >set forth in SEQ ID NO: 41, wherein Y=C or T, S= C or G and R= A or G.

According to some embodiments, the one or more TME factor comprises tumor necrosis factor alpha (TNF-α), IFN-γ, IL-6, HSP-70 or any combination thereof.

According to some embodiments, the synthetic promoter comprises additional nucleotides flanking the promoter response element and or spacing between promoter response elements.

According to some embodiments, the promoter response element comprises one or more of response element of any of IFN-γ, NF-κB, hypoxia protein, HSP-70, IL-1, IL-4, IL-6, IL-8 IL-10 response element, an IL-11 response element, an IL-12 response element, an IL-15 response element, an IL-18 response element, an IL-17 response element, an IL-21 response element, a TGF-beta response element, a GM-CSF response element, a Hepatic Growth Factor (HGF) response element, an Aryl Hydrogen Receptor (AhR) response element, a PGE2 response element (sense or anti-sense), which has been modified on one or more positions.

According to some embodiments, the modification generates a sequence with increased TME factor binding vis-à-vis the native sequence.

As a non-limiting example, the synthetic promoter may include response elements of hypoxia protein (or other TME factor response element) as derived from various hypoxia dependent target genes (LDHA/EPO/VEGF), such as the shared part of the HBS sequence of the LDHA/EPO/VEGF and/or the HAS sequence of EPO gene, as well as a linker of about 6-9 nucleotides which is not found in the target genes. This sequence is referred to as a "basic hypoxia promoter response element (PRE)", as set forth in SEQ ID NO. 42 outlined below.

>SEQ ID NO. 42:
ACGTGGCTGGAGTCACAGTCCTCTTACGTGGCTGGAGTCACAGTCCTCTT
HBS linker1 HAS linker2 HBS linker1 HBS linker2

According to some embodiments, the basic TME factor promoter (here basic hypoxia PRE) may be added at the 3' at the 5' or in the middle of the synthetic promoter sequence. According to some embodiments, the basic TME factor PRE may be inserted in a 5'-3' orientation or in a flipped 3'-5' orientation. According to some embodiments, the synthetic promoter may include a modified version of the basic TME factor PRE. A non-limiting example, of a modified basic hypoxia PRE is set forth in SEQ ID 43:
>RCGTGSCTGGAGTMACAGTCCTCTTRCGTGSCTGGAGTMACAGTC CTCTT, wherein R= A or G, S= C or G and M = A or C.

According to some embodiments, the modified TME factor PRE is the synthetic PRE that induce the less leakiness and the highest response to hypoxia stimulation.

A non-limiting example for a modified IFN-γ PRE is set forth in SEQ ID 44:
> ACTTCCSGGAARTAGGGTGGGCAAGTACTTCCSGGAART, wherein R= A or G and S= C or G.

A non-limiting example for a modified NF-κB PRE is set forth in SEQ ID 45:
>GGGGGTTTYCGGGGACTTTCCGGRRRTTTT wherein R= A or G andY = C or T.

According to some embodiments, the promoter response element comprises two or more promoter response elements; and wherein binding of TME factors to the two or more TME dependent promoter response elements induces a higher expression level of effector-genes than binding to a single TME dependent promoter response element.

According to some embodiments, the TME responsive expression vector further comprises an externally inducible promoter and a trans-activator. According to some embodiments, the synthetic promoter drives expression of the trans-activator and the externally inducible promoter drives expression of the effector-genes. According to some embodiments, the combined presence of the inducer and the TME factor induces expression of effector-genes. According to some embodiments, in the presence of the external inducer and in the absence of TME factor, essentially no effector-genes expression is detected. According to some embodiments, when the TME factor binds the promoter response element in the absence of the external inducer, essentially no effector-genes expression is detected.

According to some embodiments, the inducible promoter is a Tet-Response-Element promoter, and the external inducer is doxycycline and/or tetracycline.

According to some embodiments, the effector-gene may be, but is not limited to a CAR that comprises an antigen binding domain, a transmembrane domain, and an intracellular domain comprising a costimulatory domain and/or a primary signaling domain, wherein said antigen binding domain binds to a tumor antigen.

According to some embodiments, the CAR may be selected from the group consisting of CAR, a chemokine receptor, a cytokine receptor, a protein (or functional RNA) that enhances penetration of the immune effector cell in to the tumor, such as, but not limited to proteases of the MMP8/9, a miRNA that suppresses immuneinhibitors, such as, but not limited to PD1 and/or CTLA4, a cytokine that brings about immune-cell retention within the tumor, such as, but not limited to CXCL9/10 and/or CRCR3 ligands or any other suitable effector gene a TME which specific expression profile is desired.

According to some embodiments, the vector is selected from a DNA vector, a plasmid, a lentivirus vector, an adenoviral vector, a retrovirus vector, or other vectors for introduction of the synthetic construct into immune cells.

According to some embodiments, the TME responsive expression vector further comprises effector-genes encoding a protein (or functional RNA) that enhance penetration of the immune effector cell in to the tumor, such as, but not limited to, proteases of the MMP8/9.

According to some embodiments, the TME responsive expression vector further comprises one or more effector-genes encoding miRNAs that suppress immuneinhibitors, such as, but not limited to, PD1 and/or CTLA4, within the tumor.

According to some embodiments, the TME responsive expression vector further comprises one or more effector-genes encoding cytokines bringing about immune-cell retention within the tumor, such as, but not limited to, CXCL9/10 and/or CRCR3 ligands, thereby generating an autocrine loop.

According to some embodiments, there is provided an immune effector cell comprising the vector comprising a nucleic acid sequence encoding a synthetic promoter comprising one or more TME dependent promoter response element; and a nucleic acid sequence encoding a chimeric antigen receptor, as essentially described herein. According to some embodiments, the TME responsive vector is designed, such that binding of one or more factors present in the TME to the promoter response element directly or indirectly induces expression of the effector genes, and wherein, in the absence of TME factor binding to the promoter response element, essentially no or low/residual chimeric antigen receptor is expressed.

According to some embodiments, the immune effector cell is suitable for use as a medicament. According to some embodiments, the immune effector cell is suitable for treating a tumor of a patient in need thereof. According to some embodiments, the tumor is a solid tumor. According to some embodiments, the solid tumor is a sarcoma, a carcinomas or a lymphoma. According to some embodiments, the solid tumor is a lung tumor, melanoma, colon cancer, breast tumor or a brain tumor.

According to some embodiments, there is provided a method for treating cancer in a patient in need thereof, the method comprising administering immune cells comprising the expression vector as essentially described herein.

According to some embodiments, there is provided a method for screening a patient for determination of an optimal synthetic promoter, the method comprising obtaining a biopsy of a patient's tumor, determining the expression levels of one or more TME factors in the biopsy; and selecting a TME responsive expression vector having a TME dependent promoter response element matching the determined expression level of the one or more TME factors in the biopsy.

According to some embodiments, there is provided a method for screening a biopsy for determining an optimal synthetic promoter, the method comprising determining the expression level of one or more TME factors in the biopsy; and selecting a TME responsive expression vector having a TME dependent promoter response element matching the determined expression level of the one or more TME factors in the biopsy.

According to some embodiments, the TME may be expressed by the tumor cells and/or by non-tumor TME cells.

According to some embodiments, the method further comprises introducing the selected TME responsive expression vector into immune cells.

According to some embodiments, the immune effector cell or cell population may include, but is not limited to, T-cells and/or Natural-Killer (NK) cells.

According to some embodiments, the immune effector cell or cell population is autologous to the patient.

According to some embodiments, the immune effector cell/cell population is isolated from the patient prior to the treatment.

According to some embodiments, the method further comprises administering the immune effector cell or cell population to the patient.

Certain embodiments of the present disclosure may include some, all, or none of the above advantages. One or more technical advantages may be readily apparent to those skilled in the art from the figures, descriptions and claims included herein. Moreover, while specific advantages have been enumerated above, various embodiments may include all, some or none of the enumerated advantages.

In addition to the exemplary aspects and embodiments described above, further aspects and embodiments will become apparent by reference to the figures and by study of the following detailed descriptions.

### BRIEF DESCRIPTION OF THE FIGURES

The invention will now be described in relation to certain examples and embodiments with reference to the following illustrative figures.
**FIG. 1** schematically illustrates an expression construct comprising a synthetic promoter directly controlling reporter- and/or CAR gene expression;
**FIG. 2** schematically illustrates an expression construct comprising a synthetic promoter indirectly controlling reporter- and/or CAR gene expression;
**FIG. 3** is an illustrative flowchart of a method for screening a promoters-library for Tumor Micro-Environment (TME) providing an optimal reporter- and/or CAR gene expression;
**FIG. 4A** shows a representative histogram depicting GFP-intensity (upper panel) and percentage of GFP-positive cells (lower panel) in 293T HEK cells transduced with the expression lentiviral construct of FIG. 2 (w. GFP reporter), including the indicated TME responsive elements (G and K), in the presence and absence of TME factor and/or doxycycline;
**FIG. 4B** shows a representative FACS plot of representative replicates used for Figure 3A.
**FIG. 5** shows a representative FACS plot gating GFP-positive 293T HEK cells transfected with the expression construct of FIG. 2 (w. GFP reporter), including the indicated TME responsive elements (G and K), in the presence and absence of TME factor and/or doxycycline;
**FIG. 6** shows representative histograms quantifying the reporter intensity within the GFP-positive 293T HEK cells transfected with the expression construct of FIG. 2 (w. GFP reporter), including the indicated TME responsive elements (G and K), in the presence and absence of TME factor and/or doxycycline;
**FIG. 7** shows a representative histogram quantifying the reporter intensity within the GFP-positive 293T HEK cells transfected with the expression construct of FIG. 2 (w. GFP reporter), including the indicated TME responsive elements (G, K, J and H), in the presence and absence of TME factor and/or doxycycline;
**FIG. 8** shows exemplary FACS sorting results used to identify and sort for cells having a desired, optimal reporter gene expression profile.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

### Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "Chimeric Antigen Receptor" or alternatively a "CAR" refers to a recombinant polypeptide construct comprising at least an extracellular antigen binding domain, a transmembrane domain and a cytoplasmic signaling domain (also referred to herein as "an intracellular signaling domain") comprising a functional signaling domain derived from a stimulatory molecule as defined below. In some embodiments, the domains in the CAR polypeptide construct are in the same polypeptide chain, e.g., comprise a chimeric fusion protein. In some embodiments, the domains in the CAR polypeptide construct are not contiguous with each other, e.g., are in different polypeptide chains. According to some embodiments, the CAR may broadly refer to any moiety that is expressed by the immune cell and has a cytotoxic effect on the target cancer cell, i.e. a ligand that activates a death receptor on the target.

The terms, "tumor environment", "tumor microenvironment" and "TME" may be used interchangeably and refer to the cellular environment in which the tumor exists, including surrounding blood vessels, immune cells, fibroblasts, bone marrow-derived inflammatory cells, lymphocytes, signaling molecules and the extracellular matrix (ECM).

The term "antigen" refers to a molecule that provokes an immune response. This immune response may involve antibody production, or the activation of specific immunologically-competent cells, or both. The skilled artisan will understand that any macromolecule, including virtually all proteins or peptides, can serve as an antigen. Furthermore, one skilled in the art will understand that an antigen need not be encoded solely by a full-length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. It is readily apparent that an antigen can be generated synthesized or can be derived from a biological sample, or might be a macromolecule besides a polypeptide. Such a biological sample can include, but is not limited to, a tissue sample, a tumor sample, a cell or a fluid with other biological components.

The term "anti-tumor effect", refers to a biological effect which can be manifested by various means, including, but not limited to, e.g., a decrease in tumor volume, a decrease in the number of tumor cells, a decrease in the number of metastases, an increase in life expectancy, a decrease in tumor cell proliferation, a decrease in tumor cell survival, or amelioration of various physiological symptoms associated with the cancerous condition.

The term "autologous" refers to any material derived from the same individual to whom it is later to be re-introduced into the individual. The term "allogeneic" refers to any material derived from a different individual than to whom the material is introduced.

The term "conservative sequence modifications" refers to amino acid modifications that do not significantly affect or alter the binding characteristics of a factor thereto. Such conservative modifications include amino acid substitutions, additions and deletions.

As used herein, the term "Immune effector cell" refers to a cell that is involved in an immune response. Examples include various types, and sub-types of T cells, B cells, natural killer (NK) cells, Innate Lymphocyte Cells (ILCs), natural killer T (NKT) cells, Mast cells, Macrophage, Monocytes, Dendritic cells, Basophil, Neutrophils and Eosinophil.

The term "expression" refers to the transcription and/or translation of a particular nucleotide sequence driven by a promoter.

The term "expression vector" refers to a vector comprising a recombinant polynucleotide comprising expression control sequences operatively linked to a nucleotide sequence to be expressed. Expression vectors include all those known in the art, including cosmids, plasmids, episomes, transposons and viruses (e.g., lentiviruses, retroviruses, adenoviruses, and adeno-associated viruses) that incorporate the recombinant nucleotide sequences.

As used herein, the term "TME responsive expression vector" refers to an expression vector configured to express a gene product in the presence of factors constituting, defining or otherwise associated with a tumor environment.

As used herein, the term "promoter" refers to a DNA sequence recognized by the synthetic machinery of the cell, or introduced synthetic machinery, required to initiate the specific transcription of a polynucleotide sequence.

As used herein, the term "promoter/regulatory sequence" and "promoter response element (PRE)" may be used interchangeably and refer to nucleic acid sequences required for expression of a gene product operably linked to the promoter/regulatory sequence. As used herein, the term "TME factor" refers to a factor present and active in a TME such as but not limited to cytokines, transcription factors etc. As used herein, the term "PRE linked to a TME-associated factor refers to PRE that is activated following the excreted effect of the TME-associated factor. E.g "hypoxia PRE" refers to PRE that is activated following hypoxia in the TME. " IFN-γ PRE" refers to PRE that is activated following the presence of IFN-γ in the TME. In some instances, this sequence may be the core promoter sequence and, in other instances, this sequence may also include an enhancer sequence and other regulatory elements, which are required for expression of the gene product. The promoter/regulatory sequence may, for example, be one which expresses the gene product in a tissue specific manner.

As used herein, the term "constitutive" promoter refers to a nucleotide sequence which, when operably linked with a polynucleotide encoding a gene product, causes the gene product to be produced in a cell under most or all physiological conditions of the cell.

As used herein, the term "inducible" promoter refers to a nucleotide sequence which, when operably linked with a polynucleotide encoding a gene product, causes the gene product to be substantially enhanced only when an inducer is present. "Induction" may include both the initiation of expression from an OFF state into an ON state, as well as the enhancement of expression from relative-LOW to relative-HIGH.

As used herein, the terms "TME specific promoter", "TME inducible promoter" and "TME responsive promoter" may be used interchangeably and refer to a nucleotide sequence which causes the gene product to be induced within TME.

As used herein, the term "Synthetic promoter" refers to DNA sequences artificially synthesized as opposed to cloning of naturally occurring promoters.

The terms "cancer associated antigen" and "tumor antigen" may be used interchangeably and refer to a molecule (typically a protein, carbohydrate or lipid) that is expressed on the surface of a cancer cell, either entirely or as a fragment and which is useful for the preferential targeting of a pharmacological agent to the cancer cell. In some embodiments, a tumor antigen is a marker expressed by both normal cells and cancer cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some embodiments, a tumor antigen is a cell surface molecule that is inappropriately synthesized in the cancer cell, for instance, a molecule that contains deletions, additions or mutations in comparison to the molecule expressed on a normal cell.

As used herein, the term "treating" refers to the reduction or amelioration of the progression, severity and/or duration of a proliferative disorder, or the amelioration of one or more symptoms (preferably, one or more discernible symptoms) of a proliferative disorder resulting from the treatment. In other embodiments the term refers to the inhibition of the progression of a proliferative disorder. In other embodiments, the term refers to the reduction or stabilization of tumor size or cancerous cell count. The term "transfected" refers to a process by which an exogenous nucleic acid is transferred or introduced into a host cell. The cell includes the primary subject cell and its progeny.

The terms "specifically binds" and "binding" refer to a factor such as a transcription-factor, which recognizes and binds a cognate nucleic acid sequence.

As used herein, the terms "substantially" and "essentially" with regards to the absence of gene expression in a non-tumor environment, i.e. in healthy tissue, may include no or residual expression levels only. According to some embodiments, substantially no expression (such as in healthy tissue) may refer to expression levels at levels that are biologically/functionally ineffective against normal healthy tissues, while inducing effective levels within TME.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes sub-ranges such as 96-99%, 96-98%, 96-97%, 97-99%, 97-98% and 98-99% identity. This applies regardless of the breadth of the range.

### Description

According to some embodiments, there is provided a tumor environment (TME) responsive expression vector comprising a nucleic acid sequence encoding a synthetic promoter comprising one or more TME dependent promoter response element; and a nucleic acid sequence encoding a chimeric antigen receptor. The TME responsive vector is designed such that binding of one or more factors present in the TME to the promoter response element, directly or indirectly, induces expression of effector-genes, such as CAR.

It is understood that a trade-off may be made between including fewer response elements, so that a broad spectrum of cancers can be targeted utilizing a same TME responsive expression construct and including a more comprehensive combination of response elements increasing the specificity of the expression construct to tumor tissue as opposed to normal tissue.

According to some embodiments, the CAR is a chimeric antigen T-cell receptor (CAR-T) or a chimeric antigen Natural Killer (NK) cell receptor (CAR-NK).

According to some embodiments, the promoter response element includes/encompasses one or more interferon-gamma (IFN-γ, G)-response elements/binding sites, one or more Nuclear Factor kappa-B (NF-κB, K)-response elements/binding sites, one or more heat shock protein 70 (HSP-70) response elements/binding sites, one or more hypoxia response (H) elements/binding sites, one or more Interleukin 6 (IL-6, J) response elements/binding sites or any combination thereof. Each possibility is a separate embodiment.

According to some embodiments, the one or more TME factor may include tumor necrosis factor alpha (TNF-α), IFN-γ, IL-6, HSP-70, and/or equivalents capable of inducing similar activation pathways, or any combination thereof.

According to some embodiments, the promoter response element comprises two or more promoter response elements/binding sites. According to some embodiments, the two or more promoter response elements/binding sites may be the same or different. As a non-limiting example, the two or more promoter response elements/binding sites may include two are more NF-κB-response elements/binding sites. As another non-limiting example, the two or more promoter response elements/binding sites may include an NF-κB-response elements/binding site and an IFN-γ-response elements/binding site. According to some embodiments, binding of TME factors to the two or more TME dependent promoter response elements induces a higher expression level of CAR than binding to a single TME dependent promoter response element. As a non-limiting example, binding of NF-κB to the NF-xB-response elements/binding site and TNF-α to the IFN-γ-response elements/binding site of the promoter may, according to some embodiments, induce a higher expression of the CAR than binding of NF-κB or TNF-α alone.

According to some embodiments, the promoter response element comprises a nucleic acid selected from the group consisting of TTCCGGGAA set forth in SEQ ID NO. 1 (abbreviated herein as G), GGGAATTTCC set forth in SEQ ID NO. 2 (abbreviated herein as K), GACCTTGAGTACGTGCGTCTCTGCACGTATG set forth in SEQ ID NO. 3 (abbreviated herein as H), GCGCTTCCTGACAGTGACGCGAGCCG set forth in SEQ ID NO. 4 (abbreviated herein as J), or any combination thereof. Each possibility is a separate embodiment. As a non-limiting example, the promoter response element comprises twice the nucleic acid sequence TTCCGGGAA set forth in SEQ ID NO. 1 (abbreviated G2). As another non-limiting example, the promoter response element comprises both the nucleic acid sequence TTCCGGGAA set forth in SEQ ID NO. 1 and the nucleic acid sequence GGGAATTTCC set forth in SEQ ID NO. 2 (abbreviated G1K1). According to some embodiments, the nucleic acids may be coextensive. As a non-limiting example, the nucleic acid sequence GACCTTGAGTACGTGCGTCTCTGCACGTATG set forth in SEQ ID NO. 3 may be immediately followed by the nucleic acid sequence GCGCTTCCTGACAGTGACGCGAGCCG set forth in SEQ ID NO. 4 (abbreviated H1J1. According to some embodiments, the nucleic acids may be separated by a spacer sequence. As a non-limiting example, the nucleic acid sequence TTCCGGGAA set forth in SEQ ID NO. 1 and the nucleic acid sequence GGGAATTTCC set forth in SEQ ID NO. 2 may be spaced apart by a spacer element within the same synthetic promoter.

According to some embodiments, the TME responsive expression vector further includes a nucleic acid sequence encoding an externally inducible promoter and a nucleic acid sequence encoding a trans-activator, e.g. rtTA3. According to some embodiments, the synthetic promoter drives expression of the trans-activator and the inducible promoter drives expression of the CAR. According to some embodiments, only the combined presence of the external inducer and the TME factor results in CAR expression. According to some embodiments, the presence of the external inducer in the absence of TME factor, causes substantially no induction of CAR expression. According to some embodiments, the presence of the external inducer in the absence of TME factor, causes minimal induction of CAR expression. According to some embodiments, when the TME factor binds the promoter response element in the absence of the external inducer, essentially no CAR expression is induced. According to some embodiments, a minor level of CAR expression is also found in the un-induced state. Such minimal expression may serve to ensure that CAR-T memory is maintained.

According to some embodiments, the inducible promoter may be a Tet-Response-Element promoter, and the external inducer may be doxycycline and/or tetracycline. According to some embodiments, the Tet-Response-Element may be activated by the combined presence of the trans-activator and doxycycline and/or tetracycline. The tetracycline (Tet)-On system is an inducible gene expression system for mammalian cells, in which the reverse Tet transactivator (rtTA) fusion protein, which is composed of the doxycycline-binding Tet-repressor mutant protein and the C-terminal activator domain from the herpes simplex virus VP16 protein, is engineered to control gene expression by providing doxycycline (Dox). In the presence of Dox, rtTA activates a minimal promoter that is fused downstream of an array (e.g. seven) repeated Tet-operator sequences. Until recently, all Tet-On systems had required two separate vectors, one to introduce rtTA and another with the inducible promoter to control the gene of interest. However, a one-vector system has recently been developed, which has enabled transduction of a gene of interest into primary immune cells. By utilizing this one-vector system, it is possible to control target expression and functions using the Tet-On inducible system.

According to some embodiments, the CAR molecule encoded by the CAR sequence comprises an antigen binding domain, a transmembrane domain, and an intracellular domain, optionally comprising a costimulatory domain and/or a primary signaling domain. According to some embodiments, the antigen binding domain binds to a tumor antigen. Non-limiting examples of tumor antigens include: thyroid stimulating hormone receptor (TSHR); CD171 ; CS- 1 (CD2 subset 1 , CRACC, SLAMF7, CD319, and 19A24); C-type lectin-like molecule- 1 (CLL- 1); ganglioside GD3 (aNeu5Ac(2- 8)aNeu5Ac(2-3)bDGalp(] -4)bDGlcp(l-l )Cer); Tn antigen (Tn Ag); Fms-Like Tyrosine Kinase 3 (FLT3); CD38; CD44v6; B7H3 (CD276); KIT (CD117); Interleukin- 13 receptor subunit alpha-2 (IL- 13Ra2); Interleukin 11 receptor alpha (ILl lRa); prostate stem cell antigen (PSCA); Protease Serine 21 (PRSS21); vascular endothelial growth factor receptor 2 (VEGFR2); Lewis(Y) antigen; CD24; Platelet-derived growth factor receptor beta (PDGFR-beta); stage-specific embryonic antigen-4 (SSEA-4); Mucin 1, cell surface associated (MUC1); epidermal growth factor receptor (EGFR); neural cell adhesion molecule (NCAM); carbonic anhydrase IX (CAIX); Proteasome (Prosome, Macropain) Subunit, Beta Type, 9 (LMP2); ephrin type-A receptor 2 (EphA2); Fucosyl GM1 ; sialyl Lewis adhesion molecule (sLe); ganglioside GM3 (aNeu5Ac(2-3)bDGalp(l -4)bDGlcp(l - l)Cer; TGS5 ; high molecular weight- melanoma-associated antigen (HMWMAA); o-acetyl- GD2 ganglioside (OAcGD2); Folate receptor beta; tumor endothelial marker 1 (TEM1/CD248); tumor endothelial marker 7-related (TEM7R); claudin 6 (CLDN6); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR- 1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen- 1 (MAD-CT-1); melanoma cancer testis antigen-2 (MAD- CT-2); Fos-related antigen 1 ; p53 mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N- Acetyl glucosaminyl-transferase V (NA17); paired box protein Pax-3 (PAX3); Androgen receptor; Cyclin B 1 ; v-myc avian myelocytomatosis viral oncogene neuroblastoma derived homolog (MYCN); Ras Homolog Family Member C (RhoC); Cytochrome P450 1B 1 (CYP1B 1); CCCTC-Binding Factor (Zinc Finger Protein)-Like (BORIS); Squamous Cell Carcinoma Antigen Recognized By T Cells 3 (SART3); Paired box protein Pax-5 (PAX5); proacrosin binding protein sp32 (OY-TES 1); lymphocyte-specific protein tyrosine kinase (LCK); A kinase anchor protein 4 (AKAP-4); synovial sarcoma, X breakpoint 2 (SSX2); CD79a; CD79b; CD72; Leukocyte- associated immunoglobulin-like receptor 1 (LAIR1); Fc fragment of IgA receptor (FCAR); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor- like 2 (EMR2); lymphocyte antigen 75 (LY75); Glypican-3 (GPC3); Fc receptor-like 5 (FCRL5); and immunoglobulin lambda- like polypeptide 1 (IGLL1) and any combination thereof. Each possibility is a separate embodiment.

According to some embodiments, the TME responsive expression vector further comprises effector-genes encoding a protein (or functional RNA) that enhance penetration of the immune effector cell in to the tumor, such as, but not limited to, proteases of the MMP8/9.

According to some embodiments, the TME responsive expression vector further comprises one or more effector-genes encoding miRNAs that suppress immuneinhibitors, such as, but not limited to, PD1 and/or CTLA4, within the tumor.

According to some embodiments, the TME responsive expression vector further comprises one or more effector-genes encoding cytokines bringing about immune-cell retention within the tumor, such as, but not limited to, CXCL9/10 and/or CRCR3 ligands, thereby generating an autocrine loop.

According to some embodiments, the vector may be any suitable vector allowing expression in mammalian cells, such as human cells. According to some embodiments, the vector may be selected from a DNA vector, an RNA vector, a plasmid, a lentivirus vector, an adenoviral vector, or a retrovirus vector. Each possibility is a separate embodiment.

According to some embodiments, a TME vector library may be created, which library includes TME vectors, each having a unique TME responsive expression element profile.

According to some embodiments, there is provided an immune effector cell or cell population comprising the hereindisclosed tumor environment (TME) responsive expression vector. According to some embodiments, the immune effector cell or cell population is an NK cell or a T-cell.

According to some embodiments, there is provided a method for treating cancer in a patient in need thereof, the method comprising administering an effective amount of an immune effector cell comprising the hereindisclosed tumor environment (TME) responsive expression vector.

According to some embodiments, the method further comprises administrating to the patient an external inducer, such as, but not limited to, tetracycline and/or doxycycline. According to some embodiments, the external inducer may be provided before, concurrently with, or after the administration of the immune effector cell having the hereindisclosed tumor environment (TME) responsive expression vector.

According to some embodiments, the method may include a step of evaluating CAR expression levels and/or checking the patient for adverse effects. According to some embodiments, the CAR expression levels may be evaluated before administering the external inducer. According to some embodiments, the CAR expression levels may be evaluated, during and/or after administrating the external inducer. As a non-limiting example, a first bolus of external inducer may be initially given, followed by an evaluation of CAR expression. A second bolus may then be administered based on the CAR expression level detected and the patient's response to the treatment. According to some embodiments, the external inducer may be provided repeatedly, for example every 10 hours, every day, every two days or any other suitable time interval. According to some embodiments, the administering of the external inducer may be terminated if adverse effects are detected. According to some embodiments, the amount of external inducer administered may be increased/decreased based on the evaluated CAR expression levels and/or based on the patient's response to the treatment.

According to some embodiments, there is provided a method for screening a patient for determination of an optimal synthetic promoter for CAR expression. The method includes obtaining a biopsy of a patient's tumor, determining the expression profile of one or more TME factors in the biopsy; and selecting and/or engineering a TME responsive expression vector having a TME dependent promoter response element matching the expression profile of the one or more TME factors in the biopsy. According to some embodiments, the TME may be expressed by the tumor cells and/or by non-tumor TME cells. According to some embodiments, the non-tumor TME cells may be the immune effector cells.

According to some embodiments, a tissue sample obtained from the patient's tumor and optionally also from healthy tissue may be grown *in-vitro* and a library of TME-responsive vectors and may be used to screen for the vector proving most effective and selective for treatment, namely a vector having a TME response profile matching that of the tumor. This to obtain maximum expression in tumor tissue, while also being unique to the tumor, so that no or minimal expression is obtained in healthy tissue.

According to some embodiments, the method further includes introducing the selected TME responsive expression vector into an immune effector cell or cell population. According to some embodiments, the immune effector cell or cell population is an NK cell or a T-cell. According to some embodiments, the immune effector cell or cell population is autologous to the patient. According to some embodiments, the immune effector cell or cell population are isolated from the patient prior to the treatment.

According to some embodiments, the method further includes administering the immune effector cell or cell population to the patient.

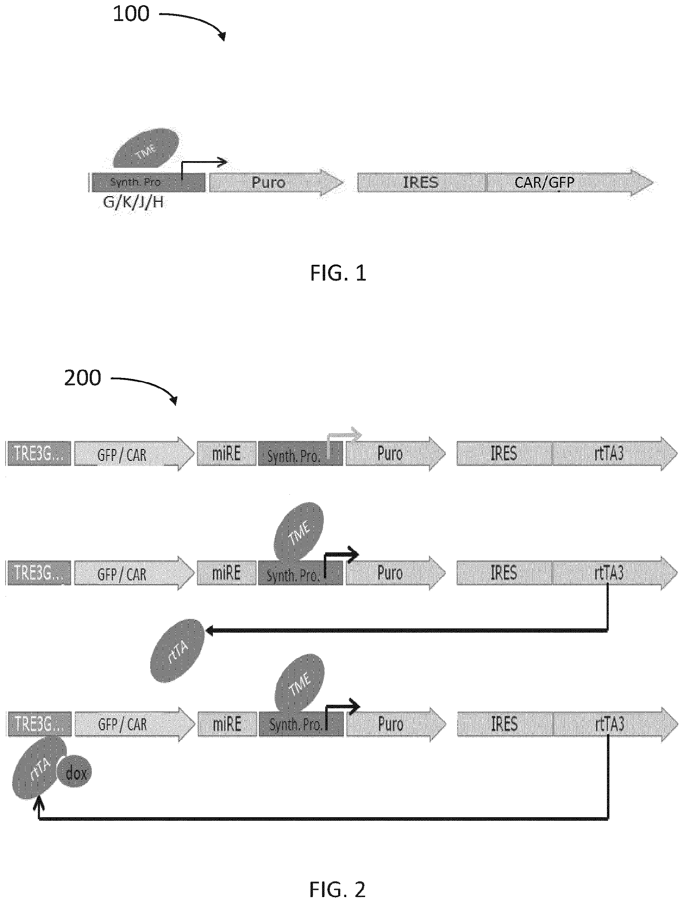

Reference is now made to **FIG. 1** which schematically illustrates an expression construct **100** comprising a synthetic promoter directly controlling expression of a CAR (or a reporter gene), according to some embodiments. Expression construct **100**, when introduced into a host immune effector cell, is configured to directly induce transcription of the CAR (or the GFP marker) in a tissue environment in which a TME factor, matching the TME response element of expression construct **100**, is prevalent.

Expression construct **100** includes the following elements:
**Synth.Pro.:** a synthetic promoter composed of (i) a minimal transcription promoter having a TATAA box that can initiate expression with adequate proximal elements; and (ii) unique combinations of promoter response elements, here IFN-γ response element (abbreviated "G"), NF-kB-response elements (abbreviated "K"), Hypoxia response Elements (abbreviated as "H"), and/or IL-6 response elements (abbreviated "J").

**TME:** marks the area of the promoter in which the promoter response element sequences are located and to which inducible factors present in the TME can bind, thereby activating the synthetic promoter (e.g. IFN-γ, TNF-α, Hypoxia and IL-6).

**Puro:** a resistance gene that is also transcribed by the synthetic promoter. The resistance gene may as here be shown to be a puromycin resistance gene; however other resistance genes are also applicable and within the scope of this disclosure. The resistance gene is introduced to enable positive *in vitro* selection of cells transduced with the vector.

**IRES:** Internal Ribosome Entry Site enables translation of both the resistance gene and the target gene (e.g. CAR or a GFP marker) from the same mRNA.

Reference is now made to **FIG. 2** which schematically illustrates an expression construct **200** comprising a synthetic promoter indirectly controlling expression of a CAR (or a reporter gene), according to some embodiments. Expression construct **200**, when introduced into a host immune effector cell, is configured to induce transcription of a transactivator in a tissue environment in which a TME factor, matching the TME response element of expression construct **200**, is prevalent. The transactivator will then induce expression of the CAR if an exogenously administered inducer (here doxycycline) is provided. Such indirect induction of CAR expression provides an ON-OFF safety mechanism, enabling cessation of CAR expression in case non-specific expression is detected or adverse effects observed.

Expression construct **200** includes the following elements:
**Synth.Pro.:** a synthetic promoter composed of (i) a minimal transcription promoter having a TATAA box that can initiate expression with adequate proximal elements; and (ii) unique combinations of promoter response elements, here IFN-γ response element (abbreviated "G"), NF-kB-response elements (abbreviated "K"), Hypoxia response Elements (abbreviated as "H"), and/or IL-6 response elements (abbreviated "J").

**TME:** marks the area of the promoter in which the promoter response element sequences are located and to which inducible factors present in the TME can bind, thereby activating the synthetic promoter (e.g. IFN-γ, TNF-α, Hypoxia and IL-6).

**Puro:** a resistance gene that is also transcribed by the synthetic promoter. The resistance gene may as here be shown to be a puromycin resistance gene; however other resistance genes are also applicable and within the scope of this disclosure. The resistance gene is introduced to enable positive *in vitro* selection of cells transduced with the vector.

**rtTA3:** trans-activator gene the transcription of which transcription is mediated by the synthetic promoter.

**IRES:** the IRES element enables transcription of both the resistance gene and the transactivator gene (here rtTA3) from the same synthetic promoter.

**Dox:** doxycycline, a potent analog of tetracycline, which serves as a co-activator to the trans-activator.

**TRE3G:** a Tet-Response-Element promoter that is activated by the combined presence of the rtTA3 and exogenous doxycycline and which mediates the transcription of CAR (or a reporter gene or other target gene). This is the basis of the enhancementsafety vector that, on the one hand, allows an amplified, TME specific CAR expression in conjunction with an additional safety layer by constructing the abovementioned synthetic promoter in front of the rtTA3 trans-activator and the reporter gene/CAR under the Tet-Response-Element promoter. The inclusion of rtTA-tet response provides an amplification of the transcription as it recruits the VP 16 transcription activator. In addition, the tetracycline/doxycycline-inducible element adds another layer of safety as it is active only in the presence of the tetracycline (or Dox), thus allowing it to turn the system OFF easily.

**miRE:** the micro-RNA element (miRE) is providing both transcription-ending for the TRE3G promoter-transcribed ORF and concurrent expression of miR for the silencing of other genes of interest (e.g. silencing of the endogenous T-cell Receptor or immune checkpoint receptors like PD-1).

According to some embodiments, there is provided a method for screening a promoter-library for having an optimal Tumor Micro-Environment (TME) expression pattern, i.e. promoters inducing strong expression in a TME, yet having little, if any, expression in non-TME.

According to some embodiments, the promoter library includes expression constructs with candidate synthetic promoters that are constructed and optionally tested functionally *in vitro.*

According to some embodiments, the promoters in the library have nucleic-acid sequences with expected binding-sites of transcription-factors that are activated within TMEs.

According to some embodiments, the promoter includes more than one candidate nucleic-acid sequence, the candidate nucleic acid sequences being spaced apart by nucleotide sequences (also referred to herein as "spacers").

According to some embodiments, the promoter further includes a minimalpromoter sequence followed by a reporter gene, such as fluorescent-proteins.

According to some embodiments, the construct includes two fluorescent reporters: a first reporter having a cryptic off-frame ATG-start codon, and a second reporter positioned after an Internal-Ribosome-Entry-Site (IRES) enabling independent translation thereof. According to some embodiments, the first fluorescent reporter will be poorly translated, yielding a signal only when highly transcribed, while the second fluorescent reporter is independently translated, also when transcribed at low-levels.

According to some embodiments, the library of candidate promoters includes promoters with a constant "core" portion, and variable nucleotides that are based on the binding-sites of the transcription factors of interest. Spacer-sequences, tandem-repeats of binding-sites, and the composition of various binding-sites of multiple factors are also enabling the generation of the libraries. According to some embodiments, the complexity may be limited by focusing variable-nucleotide on strategic positions, based on an analysis of multiple binding-sites of the transcription-factors of interest. According to some embodiments, the complexity may be increased by addition of variable nucleotides and/or repeats of binding-sites and/or the spacers between them.

As a non-limiting example, the core-sites with specific variable nucleotides may include, but not be limited to: AYTTCCSGGAART for STAT1-binding, and GGRRRTTYYC for NF-kB, where A=adenine, T=thymidine, C= cytidine, G=guanine, Y=C/T, S=C/G, R=A/G. Non-limiting examples of suitable spacers include: AGGGTGGGCAAGT, tctaga, GGGGACTTTCC.

According to some embodiments, the promoters are cloned into an expressionvector, such as, but not limited to, a pHage2 lentiviral vector with the above described first and second fluorescent-reporters, as well as additional sequences needed for plasmid propagation and for the generation of lentiviral particles.

According to some embodiments, the synthetic promoter sequences may be cloned into the expression vector using any cloning technique. Non-limiting examples of suitable cloning techniques include "classical" cloning using restriction-enzymes, and in-fusion cloning.

Optionally, PCR-amplification of the promoter library using a non-proof-reader polymerase may be utilized to introduce random mutations, which may further increase the variability and complexity of the library.

According to some embodiments, lentiviruses may be generated by common techniques, such as by transient co-transfection of the expression vector, together with packaging-plasmids, in 293T-HEK cells. Cell-lines of interest (e.g. 293 cells) may then be transduced with the virus (preferably at a MOI<0.3) to obtain a single copy per cell, and may afterwards be expanded as needed.

According to some embodiments, the method further includes identifying cells with optimal promoters, i.e. promoters inducing strong expression in a TME, yet having little if any expression in non-TME. According to some embodiments, the cells may be identified by growing the transduced cells with and without stimulus and in the presence/absence of the relevant TME factors, such as, but not limited to: TNF-alpha, IFN-gamma, Hypoxia, IL-6 and TGF-beta. The cells are then sorted (e.g. using FACS) according to their expression of the reporter genes. According to some embodiments, cells with very-high expression that gain dual-colors of both the first and the second fluorescent reporters may be separated from cells with moderate-high expression of the second reporter only.

It is understood that other techniques may be used. For example, the first and second reporters may be antibiotic resistance genes in which case the sorting may be made identifying antibiotic resistant cells.

According to some embodiments, the sorted cells are further expanded without stimulating cytokines and then sorted for negative/low expression of the first reporter in order to avoid constitutive-active promoters. Cells that show negative/low first reporter expression in the absence of stimulation are then further expanded.

According to some embodiments, the method may further include additional stimulation-sorting steps, by essentially repeating the positive and negative selections described above.

According to some embodiments, once a desired-phenotype is obtained, the method may further include extracting genomic DNA from the cells (optionally after an additional expanding of the cells) using conventional molecular biology. The promoters of the vectors are extracted using PCR; for example, using primers specific to sequences upstream and downstream of the integrated library. According to some embodiments, the primers include additional extended portions allowing direct sequencing using commercial services.

According to some embodiments, the method further includes comparing the sequences of the enriched promoters to the sequences of the library, and optionally also to known promoters to identify novel optimal promoter sequences.

According to some embodiments, the method further includes validating the activity of the identified optimal promoters in the screened cell-line and/or in other cell-lines and/or in primary immune-cells of interest.

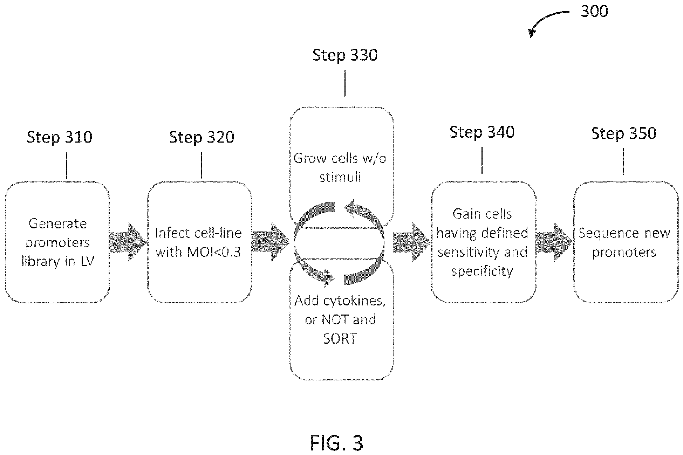

Reference is now made to **FIG. 3**, which is an illustrative flowchart **300** of the method for screening a promoters-library for providing an optimal Tumor Micro-Environment (TME) CAR-expression pattern.

In step **310** of the method a library (e.g. lentiviral (LV) library) including candidate promoters is constructed, as essentially described herein.

In step **320** cell lines (e.g. 293 cells) are infected with the lentiviruses of the library. Once cell lines expressing the promoter constructs are generated, in step 330, the cells are grown in the presence or absence of stimuli (rtTA3 and doxycycline) and in the presence or absence of stimulating cytokines and then sorted (e.g. by FACS) according to their reporter gene expression profile, by separating cells with very-high expression that gain dual-colors of both the first and the second fluorescent reporters from cells with moderate-high expression of the TME dependent reporter only. As explained herein, this step may be repeated to further enhance the sensitivity and/or specificity of the promoter.

In step **340** cells having a desired expression profile (high expression of both promoters in the presence of stimuli and cytokines, while having little or no expression of the second), TME dependent reporter, in the absence of cytokines, are identified, and their promoter sequenced (step **350**).

The following examples are presented in order to more fully illustrate some embodiments of the invention. They should in no way be construed, however, as limiting the broad scope of the invention. One skilled in the art can readily devise many variations and modifications of the principles disclosed herein without departing from the scope of the invention.

### EXAMPLES

### Example 1 - Defining response elements

Promoters, including the following response elements sequences, are listed in Table 1 below. The response elements were constructed just before a minimal transcription promoter with a TATAA box that can initiate expression with adequate proximal elements.

**Table 1. Promoter response element sequences**

| **Abbre viation** | **Response elements** | **Sequence** | **SEQ ID NO.** |
|---|---|---|---|
| K1 | 1xNF-κB | GGGAATTTCCGGGGACTTTCCGGGAATT TCCGGGGACTTTCCGGGAATTTCCAGAG CATATTAAGGTGACGCGTGTGGCCTCGA ACACCGAGCGACCCTGCAGCGACCCGC TTAAAAGCGGCCGCC | SEQ ID NO. 5 |
| G2 | 2xIFN-γ | TTCCGGGAAAGGGTGGGCAAGTTTCCG GGAAAGCAGTAGGTACAGCCTTCCGGG AAAGGGTGGGCAAGTTTCCGGGAAAGC AGTAGGTTTTTCGCATATTAAGGTGACG CGTGTGGCCTCGAACACCGAGCGACCCT GCAGCGACCCGCTTAAAAGGCGCGCC | SEQ ID NO. 6 |
| G4 | 4xIFN-γ | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGCAGTAGGT ACAGCCTTCCGGGAAAGGGTGGGCAAG TTTCCGGGAAAGCAGTAGGTTTTTCGCA TATTAAGGTGACGCGTGTGGCCTCGAAC ACCGAGCGACCCTGCAGCGACCCGCTT AAAAGGCGCGCC | SEQ ID NO. 7 |
| G6 | 6xIFN-γ | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGCAGTAGGT ACAGCCTTCCGGGAAAGGGTGGGCAAG TTTCCGGGAAAGCAGTAGGTACAGCCTT CCGGGAAAGGGTGGGCAAGTTTCCGGG AAAGCAGTAGGTACAGCCTTCCGGGAA AGGGTGGGCAAGTTTCCGGGAAAGCAG TAGGTTTTTCGCATATTAAGGTGACGCG TGTGGCCTCGAACACCGAGCGACCCTGC AGCGACCCGCTTAAAAGGCGCGCC | SEQ ID NO. 8 |
| G1K1 | 1xIFN-γ + 1xNF-κB | TTCCGGGAAAGGGTGGGCAAGTTTCCG GGAACCCGGGAATTTCCGGGGACTTTCC GGGAATTTCCGGGGACTTTCCGGGAATT TCCAGAGCATATTAAGGTGACGCGTGTG GCCTCGAACACCGAGCGACCCTGCAGC GACCCGCTTAAAAGCGGCCGCC | SEQ ID NO. 9 |
| G1K0.6 | 1xIFN-γ + 60% NF-κB | TTCCGGGAAAGGGTGGGCAAGTTTCCG GGAACCCGGGAATTTCCGGGGACTTTCC GGGAATTTCCAGAGCATATTAAGGTGA CGCGTGTGGCCTCGAACACCGAGCGAC CCTGCAGCGACCCGCTTAAAAGCGGCC GCC | SEQ ID NO. 10 |
| G2K2 | 2xIFN-γ + 2xNF-κB | TTCCGGGAAAGGGTGGGCAAGTTTCCG GGAAAGCAGTAGGTACAGCCTTCCGGG AAAGGGTGGGCAAGTTTCCGGGAAAGA GCAGGGAATTTCCGGGGACTTTCCGGG AATTTCCGGGGACTTTCCGGGAATTTCC AGAGCAGGGAATTTCCGGGGACTTTCC GGGAATTTCCGGGGACTTTCCGGGAATT TCCAGAGCATATTAAGGTGACGCGTGTG | SEQ ID NO. 11 |
| | | GCCTCGAACACCGAGCGACCCTGCAGC GACCCGCTTAAAAGGCGCGCC | |
| G3K3 | 3xIFN-γ + 3xNF-κB | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGAGCAGGGA ATTTCCGGGGACTTTCCGGGAATTTCCG GGGACTTTCCGGGAATTTCCAGAGCAG GGAATTTCCGGGGACTTTCCGGGAATTT CCGGGGACTTTCCGGGAATTTCCAGAGC AGGGAATTTCCGGGGACTTTCCGGGAAT TTCCGGGGACTTTCCGGGAATTTCCAGA GCATATTAAGGTGACGCGTGTGGCCTCG AACACCGAGCGACCCTGCAGCGACCCG CTTAAAAGGCGCGCC | SEQ ID NO. 12 |
| G3H2K 3 | 3xIFN-γ + 2xhypoxia+ 2xNF-κB | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGCAGTAGGT ACAGCCGACCTTGAGTACGTGCGTCTCT GCACGTATGAGAGCAGACCTTGAGTAC GTGCGTCTCTGCACGTATGAGAGCAGG GAATTTCCGGGGACTTTCCGGGAATTTC CGGGGACTTTCCGGGAATTTCCAGAGCA GGGAATTTCCGGGGACTTTCCGGGAATT TCCGGGGACTTTCCGGGAATTTCCAGAG CAGGGAATTTCCGGGGACTTTCCGGGA ATTTCCGGGGACTTTCCGGGAATTTCCA GAGCATATTAAGGTGACGCGTGTGGCCT CGAACACCGAGCGACCCTGCAGCGACC CGCTTAAAAGGCGCGCC | SEQ ID NO. 13 |
| G3K3H 2 | 3xIFN-γ + 3xNF-κB + 2xhypoxia | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGAGCAGGGA ATTTCCGGGGACTTTCCGGGAATTTCCG GGGACTTTCCGGGAATTTCCAGAGCAG GGAATTTCCGGGGACTTTCCGGGAATTT CCGGGGACTTTCCGGGAATTTCCAGAGC AGGGAATTTCCGGGGACTTTCCGGGAAT TTCCGGGGACTTTCCGGGAATTTCCAGA GCAGACCTTGAGTACGTGCGTCTCTGCA CGTATGAGAGCAGACCTTGAGTACGTG CGTCTCTGCACGTATGAGAGCATATTAA GGTGACGCGTGTGGCCTCGAACACCGA GCGACCCTGCAGCGACCCGCTTAAAAG GCGCGCC | SEQ ID NO. 14 |
| G2H2K 2 | 2xIFN-γ + 2xhypoxia+ 2xNF-κB | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCGACCTTGAGTACGT GCGTCTCTGCACGTATGAGAGCAGACCT TGAGTACGTGCGTCTCTGCACGTATGAG AGCAGGGAATTTCCGGGGACTTTCCGG GAATTTCCGGGGACTTTCCGGGAATTTC CAGAGCAGGGAATTTCCGGGGACTTTCC GGGAATTTCCGGGGACTTTCCGGGAATT TCCAGAGCATATTAAGGTGACGCGTGTG GCCTCGAACACCGAGCGACCCTGCAGC GACCCGCTTAAAAGGCGCGCC | SEQ ID NO. 15 |
| G2K2H 2 | 2xIFN-γ + 2xNF-κB + 2xhypoxia | TTCCGGGAAAGGGTGGGCAAGTTTCCG GGAAAGCAGTAGGTACAGCCTTCCGGG AAAGGGTGGGCAAGTTTCCGGGAAAGA | SEQ ID NO. 16 |
| | | GCAGGGAATTTCCGGGGACTTTCCGGG AATTTCCGGGGACTTTCCGGGAATTTCC AGAGCAGGGAATTTCCGGGGACTTTCC GGGAATTTCCGGGGACTTTCCGGGAATT TCCAGAGCAGACCTTGAGTACGTGCGTC TCTGCACGTATGAGAGCAGACCTTGAGT ACGTGCGTCTCTGCACGTATGAGAGCAT ATTAAGGTGACGCGTGTGGCCTCGAAC ACCGAGCGACCCTGCAGCGACCCGCTT AAAAGGCGCGCC | |
| H2G2K 2 | 2xhypoxia + 2xIFN-γ + 2xNF-κB | GACCTTGAGTACGTGCGTCTCTGCACGT ATGAGAGCAGACCTTGAGTACGTGCGT CTCTGCACGTATGAGAGCATTCCGGGAA AGGGTGGGCAAGTTTCCGGGAAAGCAG TAGGTACAGCCTTCCGGGAAAGGGTGG GCAAGTTTCCGGGAAAGAGCAGGGAAT TTCCGGGGACTTTCCGGGAATTTCCGGG GACTTTCCGGGAATTTCCAGAGCAGGG AATTTCCGGGGACTTTCCGGGAATTTCC GGGGACTTTCCGGGAATTTCCAGAGCAT ATTAAGGTGACGCGTGTGGCCTCGAAC ACCGAGCGACCCTGCAGCGACCCGCTT AAAAGGCGCGCC | SEQ ID NO. 17 |
| G1H2K 1 | 1xIFN-γ + 2xhypoxia + 1xNF-κB | TTCCGGGAAAGGGTGGGCAAGTTTCCG GGAAAGCAGTAGGTACAGCCGACCTTG AGTACGTGCGTCTCTGCACGTATGAGAG CAGACCTTGAGTACGTGCGTCTCTGCAC GTATGAGAGCAGGGAATTTCCGGGGAC TTTCCGGGAATTTCCGGGGACTTTCCGG GAATTTCCAGAGCATATTAAGGTGACGC GTGTGGCCTCGAACACCGAGCGACCCT GCAGCGACCCGCTTAAAAGGCGCGCC | SEQ ID NO. 18 |
| G1J1H 1 | 1xIFN-γ + 1xIL-6 + 1xhypoxia | TTCCGGGAAAGGGTGGGCAAGTTTCCG GGAACCCGACCTTGAGTACGTGCGTCTC TGCACGTATGTACAGCGCTTCCTGACAG TGACGCGAGCCGAGAGCATATTAAGGT GACGCGTGTGGCCTCGAACACCGAGCG ACCCTGCAGCGACCCGCTTAAAAGCGG CCGCC | SEQ ID NO. 19 |
| G1K0.6 J1 | 1xIFN-γ + 60% NF-κB + 1xIL-6 | TTCCGGGAAAGGGTGGGCAAGTTTCCG GGAACCCGGGAATTTCCGGGGACTTTCC GGGAATTTCCTACAGCGCTTCCTGACAG TGACGCGAGCCGAGAGCATATTAAGGT GACGCGTGTGGCCTCGAACACCGAGCG ACCCTGCAGCGACCCGCTTAAAAGCGG CCGCC | SEQ ID NO. 20 |
| G1K0.6 H1 | 1xIFN-γ + 60% NF-κB + 1xhypoxia | TTCCGGGAAAGGGTGGGCAAGTTTCCG GGAACCCGGGAATTTCCGGGGACTTTCC GGGAATTTCCTACAGACCTTGAGTACGT GCGTCTCTGCACGTATGAGAGCATATTA AGGTGACGCGTGTGGCCTCGAACACCG AGCGACCCTGCAGCGACCCGCTTAAAA GCGGCCGCC | SEQ ID NO. 21 |

The factors or combination of factors binding to the response elements listed in Table 1 (e.g. TNF-α, NF-κB and IL-6) are characteristic for many TME, and certain combinations of these factors may specifically represent a TME signature/profile.

However, it is noted, that the aforementioned factors may not be present in all TMEs. Similarly, additional factors may also be found in certain TMEs. Accordingly, other response elements may be included or may substitute the aforementioned response elements, and such additions and/or substitutions are within the scope of this disclosure.

Moreover, as explained herein, it is understood that a trade-off may be made between including less or more response elements. For example, including fewer types of response elements may enable targeting the expression to a broad spectrum of cancers, utilizing a same TME responsive expression construct. As another example, including a more comprehensive/exhaustive combination of response elements may increase the specificity of the expression construct to tumor tissue as opposed to normal/healthy tissue.

### Example 2 - Controlling promoter leakiness:

A major aspect of the invention is gaining an endogenous-induction of immuneeffector genes within TME. The synthetic promoter disclosed herein is configured to induce the expression within TME, while substantially limiting expression in healthy tissues. Therefore, the leakiness of the synthetic promoters was evaluated.

Two types of leakiness were envisaged.

The first type of leakiness manifests as transcription from the TME stimulated promoter, in the absence of TME factors, whether of CAR/GFP reporter, as in the case of expression construct **100** of **FIG. 1** or of the transactivator, as in the case of expression construct **200** of **FIG. 2****.** This type of leakiness may be due to induction of the minimal promoter, without an exogenously provided TME-related stimulus, either because the cells tested endogenously express the factors, or due to promoter leakiness *per se.*

The second type of leakiness level, relevant for the expression constructs including a Tet-Response-Element promoter, such as expression construct **200** of **FIG. 2**, refers to expression of CAR/GFP reporter in the absence of doxycycline/tetracycline. This type of leakiness may be due to (i) presence of residual tet/dox in media or sera used and thus be an artifact of the *in-vitro* setting; or (ii) promoter leakiness *per se.*

HEK293T cells were transduced with the expression vectors disclosed in FIG. 2 and the following response elements:
1. G2 (2xIFN-γ),
2. G1K0.6 (combination of 1XIFN-γ and 60% of NF-κB sequence element), or
3. G3K3 (combination of 3xIFN-γ and 3xNF-κB).

The cells underwent selection with puromycin to ensure the survival of only vector-transduced cells.

The GFP-intensity as well as the percentage of GFP-positive cells were tested.

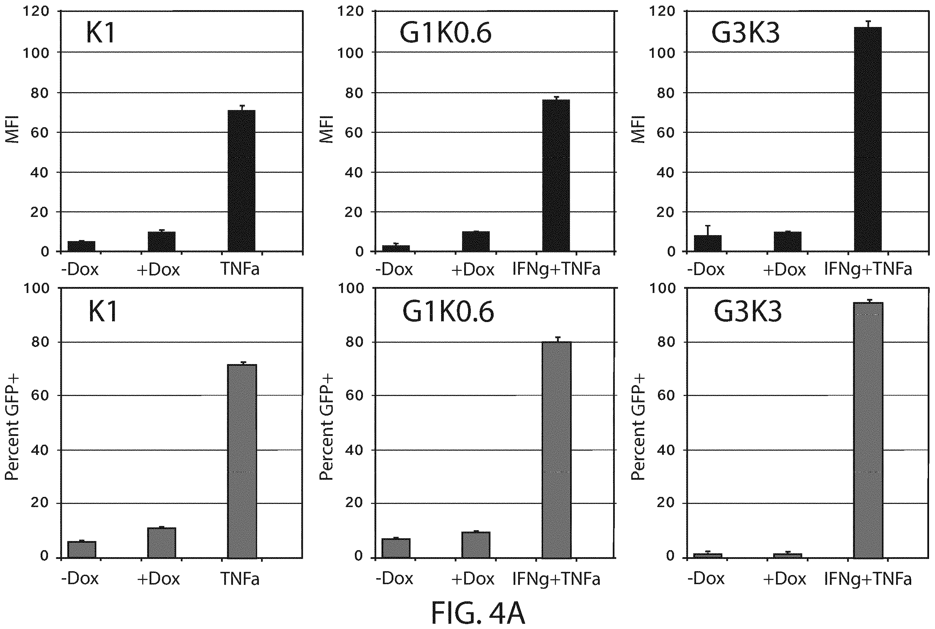

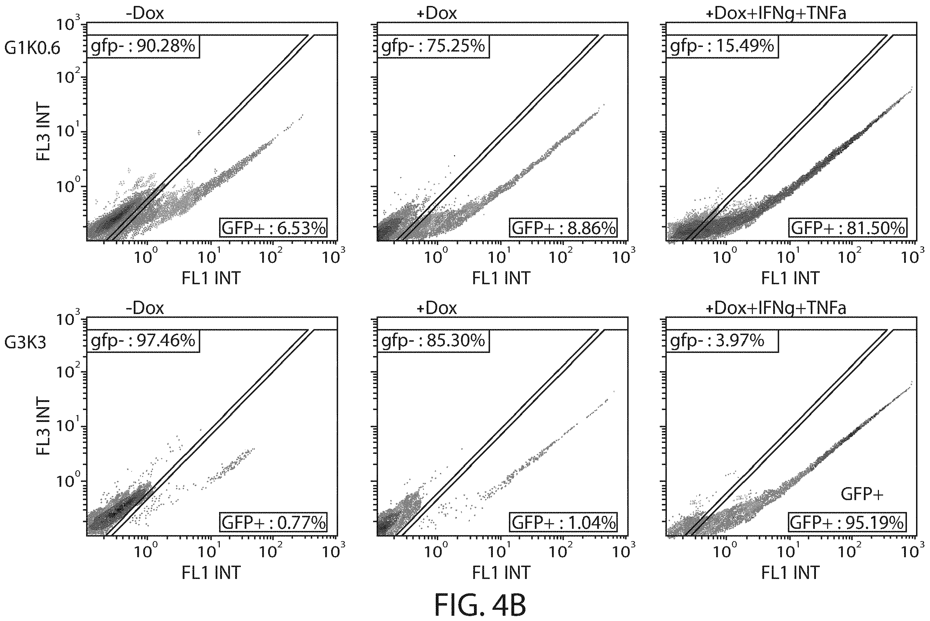

As seen from the histograms of **FIG. 4A** as well as in the representative FACS plot in **FIG. 4B**, in all three instances, adding doxycycline to the cell media did not, by itself, induce GFP expression (i.e. no increase in GFP intensity or in the percentage of GFP-positive cells was observed). However, in the combined presence of Doxycycline and TNF-α and/or IFN-γ, GFP intensity as well as the percentage of GFP-positive cells were markedly increased.

These results clearly demonstrate that the herein disclosed expression constructs enable TME specific expression.

### Example 3 - Response element synergism

It was hypothesized that including combinations of TME responsive elements would provide a synergistic expression.

The effect of including a number of IFN-γ elements (2, 4, and 6) as compared to a single IFN-γ element, reached a plateau already after two IFN-γ elements, both in the frequency of GFP-positive cells and in the GFP intensity (data not shown).

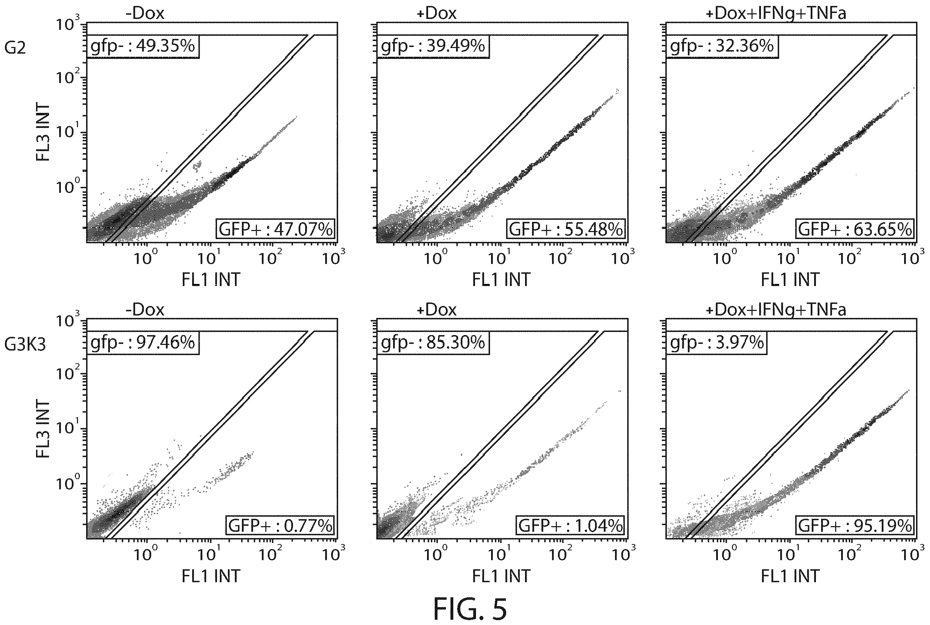

However, as seen from **FIG. 5**, a synergism was advantageously observed when NF-κB responsive elements were added to the IFN-γ elements, in that a promoter including three IFN-γ element response elements and three NF-κB response elements provided significantly higher percentages of GFP positive cells than a promoter including only IFN-γ response elements.

### Example 4 - TME profiling

Intensity and frequency of GFP expression, upon stimulus with a combination of inflammatory cytokines versus stimulus with single cytokines, was also tested. In short, HEK293T cells were transduced with vectors containing one of the following synthetic promoters:
1. G1K0.6: 1xIFN-γ and 60% of NF-κB,
2. G3K3: combination of 3xIFN-γ and 3xNF-κB.

This time the cells were harvested without prior puromycin selection, and the results thus represent the entire cell population, transduced as well as non-transduced cells.

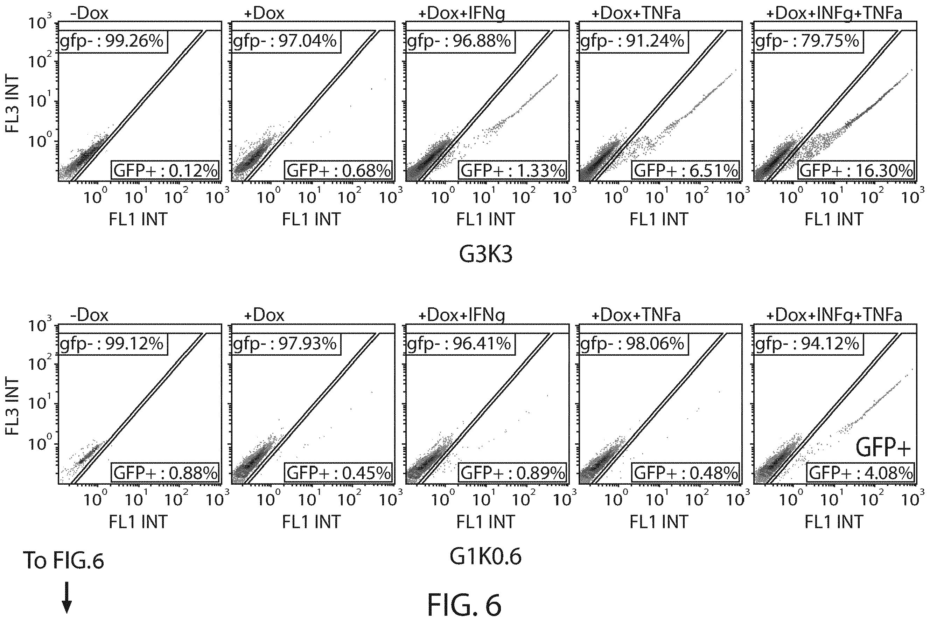

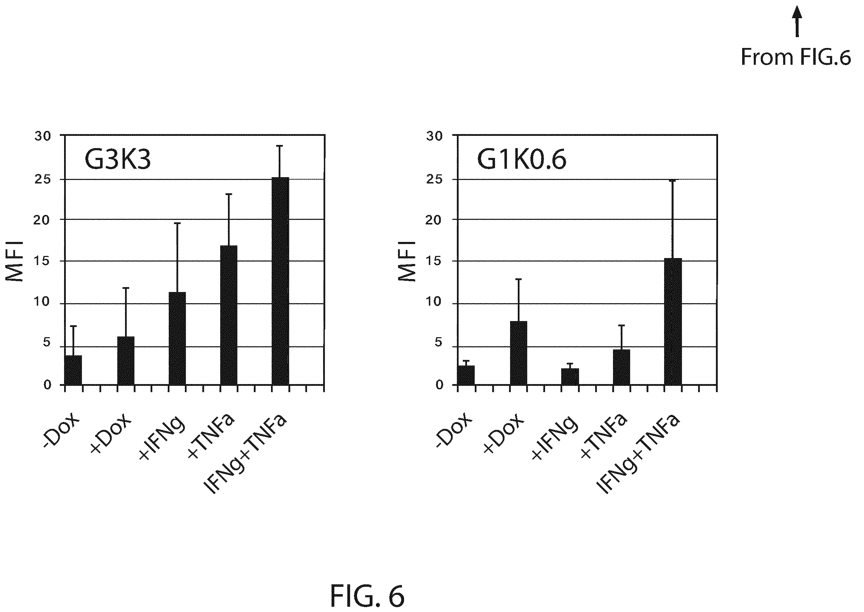

**FIG. 6** shows representative FACS plots (upper panels), gating GFP-positive cells (GFP+), as well as histograms (lower panels) showing a quantification of the GFP intensity (Median) for each tested condition.

Notably, transducing cells with the expression construct, having a G3K3 response element, showed cumulative expression, per cytokine added. In cells transduced with the expression construct having a G1K0.6 response element, adding each of IFN-γ and TNF-α separately caused only low levels of expression, whereas combined treatment with both IFN-γ and TNF-α induced substantial higher expression levels.

These results clearly show that controlled levels of expression can be achieved using the hereindisclosed expression constructs. Preferably, the construct utilized should be accustomed to the TME profile of the cells treated/targeted so as to optimize the expression level based on need.

### Example 5 - 3-factor response element

Following the study with combinations of two response elements IFN-γ and NF-κB (G and K), response elements including binding sites of additional factors, namely IL-6 and hypoxia (J and H), were also evaluated essentially as described above.

HEK293T cells were transduced with vectors containing one of the following synthetic promoters:
1. G1K0.6J1: 1XIFN-γ, 60% of NF-κB, 1xIL-6 response element sequences.
2. G1K0.6H1: 1XIFN-γ, 60% of NF-κB, 1xHypoxia response element sequences.

GFP intensity and frequency of expression, was evaluated by FACS before and after stimulation with the indicated factors. Cells were harvested without selection.

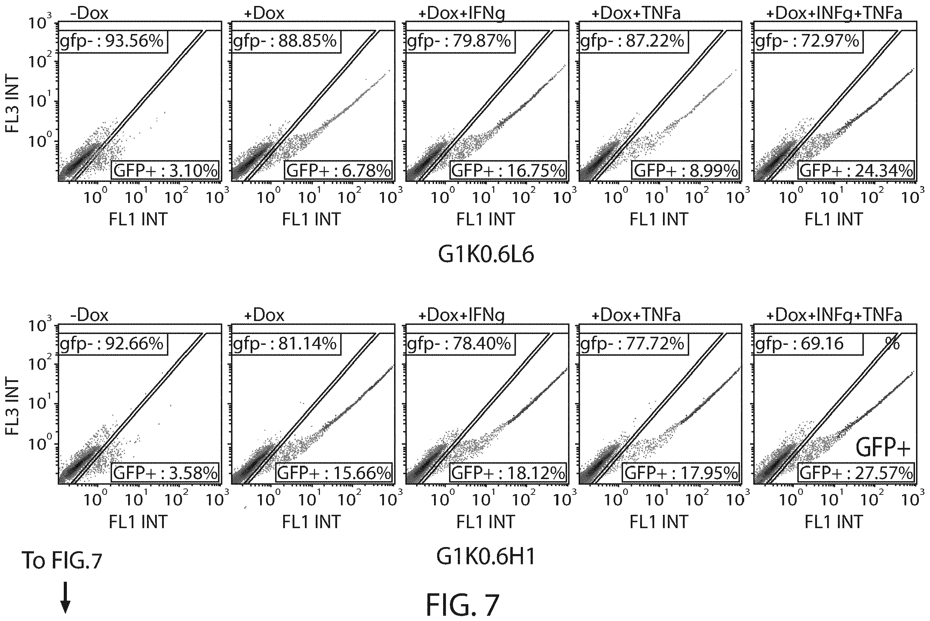

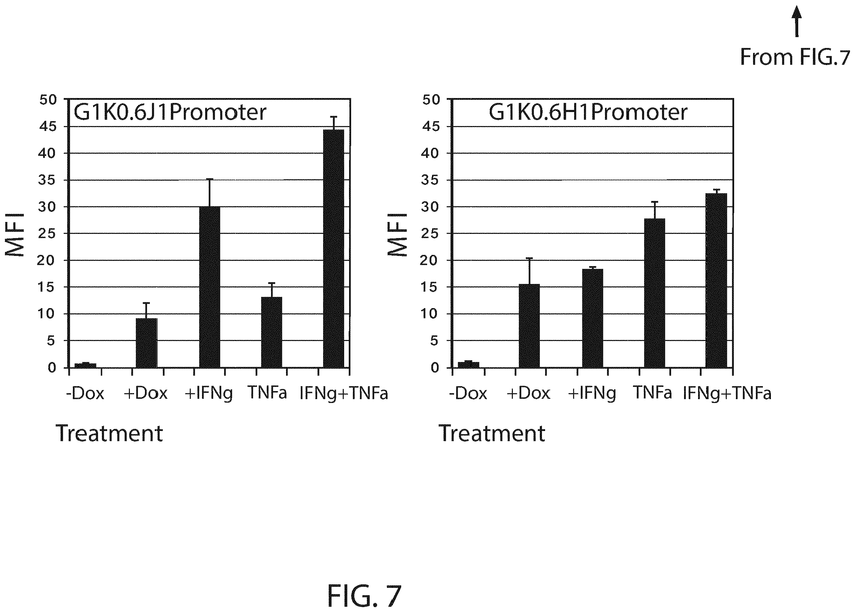

**FIG. 7** shows FACS plots (upper panels) of GFP-positive cells (GFP+) for each promoter and on each condition and histogram (lower panels) depicting quantification of the expression intensity (as Median) of the GFP-positive cells at each condition.

As seen from **FIG. 7**, the insertion of a third response element downstream to G1K0.6 elements did not further contribute to its synergistic effect. It is noted that this may not necessarily be a general observation but rather be representative of the specific cell line tested.

### Example 6 - screening for optimal promoter sequences

In order to identify promoters providing an optimal CAR expression profile, a lentiviral library of candidate promoters was constructed. The library includes constructs with promoters having a constant "core" portion, and variable nucleotides that are based on the binding-sites of the transcription factors of interest. Spacer-sequences, tandem-repeats of binding-sites, and the composition of various binding-sites of multiple factors are also included. The variability of the promoters was generated by changing, adding and/or deleting nucleotides at strategic and/or random positions of the promoters. Complexity was further increased by varying the number of binding-site repeats and/or the spacers between them. A total of 65,536 promoter sequences were included in the screen.

The constructs of the library include a fluorescent reporter (GFP) having a cryptic off-frame ATG-start codon ensuring that the reporter gene is only translated in the presence of high level of transcripts, i.e. high levels of transcription factor, here TME transcription factors. The constructs also include a second reporter (DsRed) positioned after an Internal-Ribosome-Entry-Site (IRES) enabling independent translation thereof, also at low levels of transcripts.

The constructs were then cloned into lentiviral vectors, and viruses were generated by transient co-transfection of the lentiviral vector together with packaging-plasmids into 293T-HEK cells.

293 cells were then infected (transduced) with the lentiviruses to obtain cell lines having incorporated into their genome a promoter construct of the library.

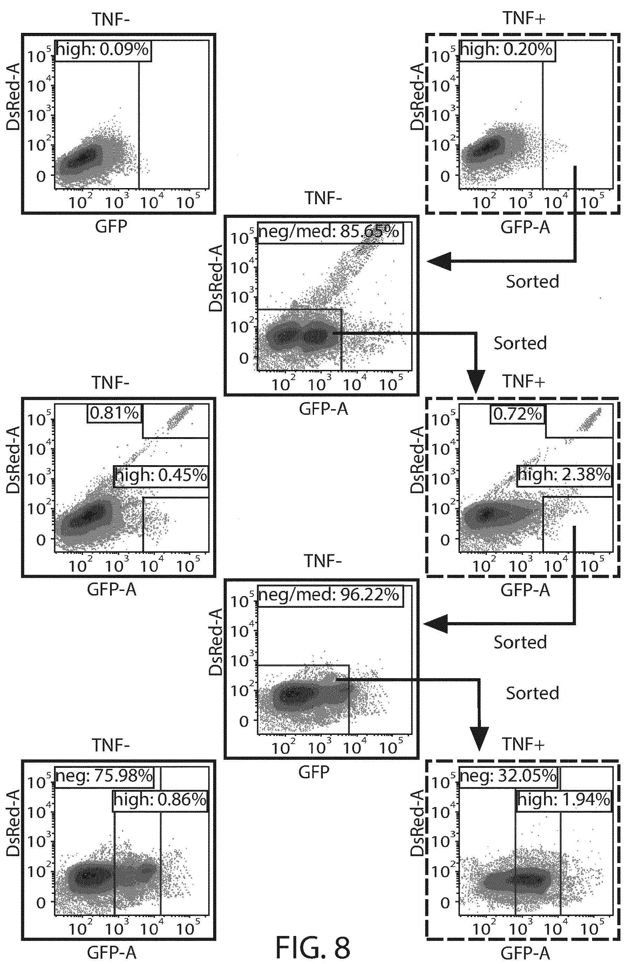

Subsequently, the transduced cells were then grown for 48h in the presence or absence of 250U/mL TNF, IFN or TNF and IFN and subsequently subjected to FACS sorting. Initially, cells showing high GFP expression or high GFP and DsRed expression in the presence of TNF, IFN or TNF and IFN were gated ("positive" sorting). As seen from the upper panel of **FIG. 8**, in the absence of cytokines, only 0.09% of the cells showed high GFP expression, while in the presence of TNF, IFN or TNF and IFN 0.2% of the cells had high GFP expression.

The gated cells were then subjected to a round of "negative" sorting after having been grown in the absence of cytokines (**FIG. 8** second panel). During this sorting step, cells having no or little GFP expression in the absence of cytokines were acquired, whereas cells with promoters causing constitutive expression were discarded (or separately sorted).

The positive and negative rounds of sorting were repeated twice (**FIG. 8** 3^{rd} and 4^{th} panels) until a final population, being 1.94% of the initial population, was acquired, which population being characterized by having high GFP expression, i.e. having an expression pattern highly sensitive to the presence/absence of TNF and/or IFN.

The acquired cells were propagated, and their promoter was sequenced using promoter specific primers. The screen identified 19 sequences out of the 65,536 sequences included in the screen. 18 were essentially similar but differed in 16 nucleotides (bold and underlined) plus in some instances some additional more random changes. An additional sequence (SEQ ID NO: 41) somewhat different from the other 18 was also retrieved
Table 2 below provides the sequences of promoters providing an optimal CAR expression profile, retrieved from the screen.

**Table 2 - promoter sequences**

| **SEQ ID NO.** | **Sequence** |
|---|---|
| 22 | TACAGGGACAGCAGAGATCCAGTTTGGACTAGCCCGGTCGCAC TAGTTCTAGAACTTCCCGGAAATAGGGTGGGCAAGTATTTCCG GGAAATTCTAGAGGGAGTTCCCGGGGACTTTCCGGGGATTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 23 | TACAGGGACAGCAGAGATCCAGTTTGGACTAGCCCGGTCGCAC TAGTTCTAGAACTTCCCGGAAGTAGGGTGGGCAAGTACTTCCC GGAAGTTCTAGAGGAAATTTTCGGGGACTTTCCGGGGGTTCTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 24 | TACAGGGACAGCAGAGATCCAGTTTGGACTAGCCCGGTCGCAC TAGTTCTAGAACTTCCCGGAAGTAGGGTGGGCAAGTACTTCCG GGAAATTCTAGAGGGAGTTCTCGGGGACTTTCCGGGAATTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 25 | TACAGGGACAGCAGAGATCCAGTTTGGACTAGCCCGGTCGCAC TAGTTCTAGAACTTCCCGGAAGTAGGGTGGGCAAGTATTTCCC GGAAGTTCTAGAGGAAGTTCTCGGGGACTTTCCGGAGATTCTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 26 | TACAGGGACAGCAGAGATCCAGTTTGGACTAGCCCGGTCGCAC TAGTTCTAGAACTTCCGGGAAATAGGGTGGGCAAGTACTTCCG GGAAATTCTAGAGGGGATTTCCGGGGACTTTCCGGAGGTTCTC TCTAGATATTAAGGTGACGCGTGTGGCCTCGAACACCGAGCGA |
| | CCCTGCAGCGACCCGCTTAAAAGCGGCCGCCATGGGCCGCCAT GGCCTCCTCCGAGGACGTCATCAAGGAGTTCATGC |
| 27 | TACAGGGACAGCAGAGATCCAGTTTGGACTAGCCCGGTCGCAC TAGTTCTAGAACTTCCGGGAAATAGGGTGGGCAAGTACTTCCG GGAAGTTCTAGAGGAGGTTTTCGGGGACTTTCCGGAGGTTTCC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 28 | TACAGGGACAGCAGAGATCCAGTTTGGACTAGCCCGGTCGCAC TAGTTCTAGAACTTCCGGGAAGTAGGGTGGGCAAGTATTTCCC GGAAGTTCTAGAGGGGGTTTTCGGGGACTTTCCGGAGGTTTCC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 29 | TACAGGGACAGCAGAGATCCAGTTTGGACTAGCCCGGTCGCAC TAGTTCTAGAACTTCCGGGAAGTAGGGTGGGCAAGTATTTCCG GGAAATTCTAGAGGGAGTTCTCGGGGACTTTCCGGGGATTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 30 | TACAGGGACAGCAGAGATCCAGTTTGGACTAGCCCGGTCGCAC TAGTTCTAGAATTTCCCGGAAATAGGGTGGGCAAGTACTTCCG GGAAATTCTAGAGGGGGTTTCCGGGGACTTTCCGGGGGTTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 31 | TACAGGGACAGCAGAGATCCAGTTTGGACTAGCCCGGTCGCAC TAGTTCTAGAATTTCCCGGAAATAGGGTGGGCAAGTATTTCCC GGAAATTCTAGAGGGGGTTTTCGGGGACTTTCCGGGAATTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 32 | TACAGGGACAGCAGAGATCCAGTTTGGACTAGCCCGGTCGCAC TAGTTCTAGAATTTCCCGGAAATAGGGTGGGCAAGTATTTCCG GGAAATTCTAGAGGAGGTTCTCGGGGACTTTCCGGGAGTTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 33 | TACAGGGACAGCAGAGATCCAGTTTGGACTAGCCCGGTCGCAC TAGTTCTAGAATTTCCCGGAAATAGGGTGGGCAAGTATTTCCG GGAAATTCTAGAGGAGGTTTTCGGGGACTTTCCGGGAGTTTCC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 34 | TACAGGGACAGCAGAGATCCAGTTTGGACTAGCCCGGTCGCAC TAGTTCTAGAATTTCCCGGAAATAGGGTGGGCAAGTATTTCCG GGAAGTTCTAGAGGAAGTTTTCGGGGACTTTCCGGGAATTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 35 | TACAGGGACAGCAGAGATCCAGTTTGGACTAGCCCGGTCGCAC TAGTTCTAGAATTTCCGGGAAATAGGGTGGGCAAGTATTTCCC GGAAGTTCTAGAGGAGATTCTCGGGGACTTTCCGGGGGTTCTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 36 | TACAGGGACAGCAGAGATCCAGTTTGGACTAGCCCGGTCGCAC TAGTTCTAGAATTTCCGGGAAGTAGGGTGGGCAAGTACTTCCG GGAAATTCTAGAGGGGGTTTCCGGGGACTTTCCGGAGATTCTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 37 | TACAGGGACAGCAGAGATCCAGTTTGGACTAGCCCGGTCGCAC TAGTTCTAGAATTTCCGGGAAATAGGGTGGGCAAGTATTTCCC GGAAGTTCTAGAGGGGGTTTTCGGGGACTTTCCGGAAATTTTC |
| | TCTAGATATTAAGGTGACGCGTGTGGCCTCGAACACCGAGCGA CCCTGCAGCGACCCGCTTAAAAGCGGCCGCCATGGGCCCCATG GCCTCCTCCGAGGACGTCATCAAGGAGTTCATGC |
| 38 | TACAGGGACAGCAGAGATCCAGTTTGGACTAGCCCGGTCGCAC TAGTTCTAGAACTTCCCGGAAATAGGGTGGGCAAGTATTTCCC GGAAGTTCTAGAGGAGGTTTTCGGGGACTTTCCGGGATTCCCT CTAGATATTAAGGTGACGCGTGTGGCCTCGAACACCGAGCGAC CCTGCAGCGACCCGCTTAAAAGCGGCCGCCATGGGCCGCCATG GCCTCCTCCGAGGACGTCATCAAGGAGTTCATGC |
| 39 | TACAGGGACAGCAGAGATCCAGTTTGGACTAGCCCGGTCGCAC TAGTTCTAGTTCCGGGAAGTGGGTGGGCAATATTTCCCGGAAG TTTAGAGGAAGTTTTCGGGGACTTCCGGAAATTCCCTCTAGAT ATTAAGGTGACGCGTGTGGCCTCGAACACCGAGCGACCCTGCA GCGACCCGCTTAAAAGCGGCCGCCATGGGCCGCCATGGCCTCC TCCGAGGACGTCATCAAGGAGTTCATGC |
| 40 | TACAGGGACAGCAGAGATCCAGTTTGGACTAGCCCGGTCGCAC TAGTTCTAGAACTTCTCGGAAATAGGGTGGGCAAGTACTGTGG CCTCGAACACCGAGCGACCCTGCAGCGACCCGCTTAAAAGCGG CCGCCATGGGCCGCCATGGCCTCCTCCGAGGACGTCATCAAGG AGTTCATGCAGACCAAGTCTCTGCTACC |

### Example 7 - Designing new PRE-based libraries and combining basic sequence or library-optimized sequence with other PREs into the same synthetic promoter

To generate a library of a specific PRE, either alone or combined with other PREs we took the following general approach, here described in detail while using the hypoxia PRE element as an example.

PRE sequences utilized in hypoxia dependent promoters of different genes were collected.

Based on the sequences a basic, non-naturally occurring hypoxia PRE element was generated by taking portions from natural occurring sequences suggested to function as hypoxia PRE in different genes. The portions include:

HBS sequence (ACGTG) found for example in the hypoxia dependent LDHA/EPO/VEGF genes.

Linker 1 (GCTGGAGT) an 8-nucleotide linker, not found in the promoters of natural target genes (not part of LDHA/EPO/VEGF genes).

HAS (CACAG) found for example in the hypoxia dependent EPO gene.

Linker 2 (TCCTCTT) a 7-nucleotide linker, not found in the promoters of natural target genes (not part of LDHA/EPO/VEGF genes).

These sequences were linearly combined into one sequence and then doubled in tandem to obtain the sequence set forth in SEQ ID. NO. 42

>ACGTGGCTGGAGTCACAGTCCTCTTACGTGGCTGGAGTCAC
AGTCCTCTT

Representing a "basic hypoxia PRE". It is understood that the basic hypoxia PRE is exemplary only and that other combinations, linkers, number of PREs as well as their orientations can also be envisaged.

The "basic hypoxia sequence" can then be added to additional PRE sequences (of the same or a different TME) to generate modified/alternative synthetic promoters. Non-limiting examples of optional modified/alternative synthetic promoters based on a basic TME factor (here hypoxia) PRE include
(i) Single or combined PREs,
   As a non-limiting example, the basic hypoxia PRE may be combined with G1K1 sequence (set forth in SEQ ID NO. 9), thereby generating the sequence set forth in SEQ ID NO. 46 (also referred to as H1G1K1). 
>ACGTGGCTGGAGTCACAGTCCTCTTACGTGGCTGGAGTCAC
AGTCCTCTTACTAGTTCTAGAACTTCCCGGAAGTAGGGTGGG
CAAGTACTTCCCGGAAGTTCTAGAGGAAATTTTCGGGGACTT
TCCGGGGGTTCTCTCTAGATATTAAGGTGACGCGTGTGGCCT
CGAACACCGAGCGACCCTGCAGCGACCCGCTTAAAAGCGGC
 
CGCCATGGGCCGCCATGGCCTCCTCCGAGGACGTCATCAAG
GAGTTC
(ii) Alternative positioning: the basic TME factor PRE can be added 5 prime and/or 3 prime and/or the middle of the synthetic promoter.
(ii) Alternative orientation: the basic TME factor PRE can be flipped and added as above in (i) and (ii).
   As a non-limiting example the basic hypoxia PRE may be combined with G1K1 sequence (set forth in SEQ ID NO. 9) in a flipped configuration thereby generating the sequence set forth in SEQ ID NO. 47 (also referred to as H1flipG1K1. 
>AAGAGGACTGTGACTCCAGCCACGTAAGAGGACTGTGACT
CCAGCCACGTACTAGTTCTAGAACTTCCCGGAAGTAGGGTG
GGCAAGTACTTCCCGGAAGTTCTAGAGGAAATTTTCGGGGA
CTTTCCGGGGGTTCTCTCTAGATATTAAGGTGACGCGTGTGG
CCTCGAACACCGAGCGACCCTGCAGCGACCCGCTTAAAAGC
GGCCGCCATGGGCCGCCATGGCCTCCTCCGAGGACGTCATC
AAGGAGTTC
(iv) Alternative Sequence. Nucleotides of the basic TME factor PRE sequence can be substituted. For example, as set forth in SEQ ID NO. 43.

>RCGTGSCTGGAGTMACAGTCCTCTTRCGTGSCTGGAGTMAC
AGTCCTCTT

Following the generation of the library, the library screen for the best sequences; i.e. the sequences that induce the less leakiness and the highest response to hypoxia stimulation, as essentially described in Example 6.

It is understood that the described approach for designing "basic response elements" and then generating a library of modified/alternative PREs based on the "basic response element" - may be adapted for all of the herein described TME factor PREs.

While certain embodiments of the invention have been illustrated and described, it will be clear that the invention is not limited to the embodiments described herein. Numerous modifications, changes, variations, substitutions and equivalents will be apparent to those skilled in the art without departing from the spirit and scope of the present invention as described by the claims, which follow.

### List of itemized embodiments

1. A Tumor Micro-Environment (TME) responsive expression vector comprising: a nucleic acid sequence encoding a synthetic promoter, said promoter comprising one or more TME dependent promoter response elements (PRE); and a nucleic acid sequence encoding an effector gene; wherein said TME responsive expression vector is designed, such that binding of one or more TME factors present in the TME to the promoter response element induces expression of the effector-gene, and wherein, in the absence of binding of the one or more TME factor to the promoter response element, low or essentially no effector gene is expressed.
2. The TME responsive expression vector of embodiment 1, wherein the TME dependent PRE comprises one or more sequence elements selected from the list consisting of: interferon-gamma- (IFN-γ) PRE, TNF-alpha PRE, Nuclear Factor kappa-B (NF-κB) PRE, hypoxia PRE, Heat shock protein 70 (HSP-70) PRE, IL-6 TGF-beta PRE, IL-1 PRE, IL-8 PRE, IL-11 PRE, IL-12 PRE, IL-15 PRE, IL-18 PRE, IL-17 PRE, IL-21 PRE,IL-35 PRE, GM-CSF PRE, Hepatic Growth Factor (HGF) PRE, Aryl Hydrogen Receptor (AhR) PRE or any combination thereof, activated within an inflammatory TME.
3. The TME responsive expression vector of embodiment 1 or 2, wherein the effector gene is a chimeric antigen receptor (CAR).
4. The TME responsive expression vector of any of the preceding embodiments, wherein the promoter response element comprises a nucleic acid sequence with at least having at least 80% sequence homology to a nucleic acid selected from the nucleic acid sequences set forth in SEQ ID Nos 1-40 or any combination thereof.
5. The TME responsive expression vector of any of the preceding embodiments, wherein the promoter response element comprises a nucleic acid sequence with at least having at least 80% sequence homology to a nucleic acid sequence selected from the nucleic acid sequences set forth in SEQ ID Nos 1-4.
6. The TME responsive expression vector of any of the preceding embodiments, wherein the promoter response element comprises a nucleic acid sequence having at least 80% sequence homology to a nucleic acid sequence selected from the nucleic acid sequences set forth in SEQ ID Nos 22-40 or any combination thereof.
7. The TME responsive expression vector of embodiment 1, wherein the promoter response element comprises a nucleic acid sequence with at least having at least 80% sequence homology to the nucleic acid set forth in SEQ ID NO: 41.
8. The TME responsive expression vector of any of embodiments 1-7, wherein the promoter response element comprises two or more promoter response elements; and wherein binding of the one or more TME factors to the two or more TME dependent promoter response elements induces a higher expression level of CAR than when binding to a single TME dependent promoter response element.
9. The TME responsive expression vector of any of embodiments 1-8, further comprising an externally inducible promoter and a trans-activator; wherein the synthetic promoter drives expression of the trans-activator and wherein the externally inducible promoter drives expression of the CAR; and wherein combined presence of the inducer and the TME factor induces expression of CAR.
10. The TME responsive expression vector of embodiment 9, wherein, in the presence of the external inducer and in the absence of TME factor, essentially no CAR expression is detected.
11. The TME responsive expression vector of embodiment 9 or 10, wherein, when the TME factor binds the promoter response element in the absence of the external inducer, essentially no CAR expression is detected.
12. The TME responsive expression vector of any of embodiments 9-11, wherein the externally inducible promoter is a Tet-Response-Element promoter and the external inducer is doxycycline and/or tetracycline, and wherein the Tet-Response-Element is activated by the combined presence of the trans-activator and doxycycline and/or tetracycline.
13. The TME responsive expression vector of embodiment 12, wherein the transactivator is rtTA3.
14. The TME responsive expression vector of any of embodiments 1-13, wherein the CAR molecule encoded by the CAR sequence comprises an antigen binding domain, a transmembrane domain, and an intracellular domain comprising a costimulatory domain and/or a primary signaling domain, wherein said antigen binding domain binds to a disease associated tumor antigen.
15. The TME responsive expression vector of any of embodiments 1-14, wherein the vector is selected from a DNA vector, a plasmid, a lentivirus vector, an adenoviral vector, or a retrovirus vector.
16. An immune effector cell comprising the TME responsive expression vector of any of embodiments 1-15.
17. The immune effector cell of embodiment 16 for use as a medicament.
18. The immune effector cell of embodiment 16 for treating a tumor of a patient in need thereof.
19. The immune effector cell of embodiment 18, wherein the tumor is a solid tumor.
20. The immune effector cell of embodiment 19, wherein the solid tumor is a sarcoma, a carcinomas or a lymphoma.
21. The immune effector cell of embodiment 20, wherein the solid tumor is a lung tumor, melanoma, colon cancer, breast tumor or a brain tumor.
22. A method for screening a tumor biopsy for determination of an optimal synthetic promoter, the method comprising determining the expression levels of one or more TME factors in the biopsy; and selecting a TME responsive expression vector having a TME-dependent promoter response element matching a TME of the patient.
23. The method of embodiment 22 further comprising, introducing the selected TME responsive expression vector into an immune effector cell or cell population.
24. The method of embodiment 23, wherein the immune effector cell or cell population is an NK cell or a T-cell.
25. The method of embodiment 23, wherein the immune effector cell or cell population is autologous to the patient.
26. The method of embodiment 23, wherein the immune effector cell or cell population are immune effector cell or cell population isolated from a patient prior to the treatment.
27. The method of embodiment 23, wherein the one or more TME factors is expressed by the tumor and/or by non-tumor TME cells.
28. The method of embodiment 23, wherein the selecting of the TME responsive expression vector having a TME-dependent promoter response element matching the TME of the patient comprises synthesizing the TME-dependent promoter response element.
29. The method of embodiment 23, wherein the selecting of the TME responsive expression vector having a TME-dependent promoter response element matching the TME of the patient comprises choosing a TME-dependent promoter response element from a library of TME-dependent promoter response element best matching the TME of the patient.

## Claims

1. A Tumor Micro-Environment (TME) responsive expression vector comprising:
a nucleic acid sequence encoding an effector gene operably linked to a synthetic promoter, said promoter comprising two or more different TME dependent promoter response elements (PRE)s selected from interferon-gamma-(IFN-γ) PRE, NPκB PRE, TNF-alpha PRE, TGFβ PRE, hypoxia PRE, IL-6 PRE, heat shock protein 70 (HSP-70) PRE, IL-1 PRE, IL-8 PRE, IL-11 PRE, IL-12 PRE, IL-15 PRE, IL-18 PRE, IL-17 PRE, IL-21 PRE,IL-35 PRE, GM-CSF PRE, hepatic growth factor (HGF) PRE, and aryl hydrogen receptor (AhR) PRE;
wherein the TME responsive expression vector is so constructed that:
a) the presence in the TME of at least two TME factors which activate the two or more PREs induces a higher expression level of the effector gene than when only one of the TME factors is present in the TME,
and
b) minimal or low expression of the effector gene occurs in the absence of the TME factors which activate the two or more PREs,
so that the effector gene is upregulated and directing activities against the tumor while sparing normal tissue.

2. The TME responsive expression vector of claim 1, wherein the two or more different PREs are selected from interferon-gamma-(IFN-γ) PRE, NPκB PRE, TNF-alpha PRE, TGFβ PRE, hypoxia PRE, and IL-6 PRE.

3. The TME responsive expression vector of claim 2, wherein the two or more different PREs comprise TGFβ PRE, and at least one of hypoxia PRE, NPκB PRE, and IL-6 PRE.

4. The TME responsive expression vector of claim 2, wherein the two or more different PREs comprise interferon-gamma-(IFN-γ) PRE, NPκB PRE, and IL-6 PRE or hypoxia PRE.

5. The TME responsive expression vector of claim 2, wherein the two or more different PREs comprise interferon-gamma-(IFN-γ) PRE, IL-6 PRE, and hypoxia PRE.

6. The TME responsive expression vector of any one of claims 1-5, wherein no more than two PREs can be bound by one TME factor.

7. The TME responsive expression vector of any of claims 1-6, further comprising an externally inducible promoter and a trans-activator; wherein the synthetic promoter drives expression of the trans-activator and wherein the externally inducible promoter drives expression of the effector gene; and wherein combined presence of an external inducer of the externally inducible promoter and of the TME factors induces expression of the effector gene.

8. The TME responsive expression vector of claim 7, wherein the externally inducible promoter is a Tet-Response-Element promoter, and the external inducer is doxycycline and/or tetracycline, and wherein the Tet-Response-Element is activated by the combined presence of the trans-activator and doxycycline and/or tetracycline; preferably wherein the trans-activator is rtTA3.

9. The TME responsive expression vector of any one of claims 1-8, wherein the effector gene is selected from a chimeric antigen receptor (CAR), a chemokine receptor, a cytokine receptor, an MMP, a protein or functional RNA that enhance penetration of an immune effector cell into a tumor, a miRNA that suppresses immune-inhibitors, a cytokine that brings about immune-cell retention within the tumor, and a CRCR3 ligand.

10. The TME responsive expression vector of claim 9, wherein the effector gene is a CAR.

11. The TME responsive expression vector of claim 10, wherein the CAR comprises an antigen binding domain, a transmembrane domain, and an intracellular domain comprising a costimulatory domain and/or a primary signalling domain, wherein said antigen binding domain is capable of binding to a disease associated tumor antigen.

12. An immune effector cell comprising the TME responsive expression vector of any of claims 1-11.

13. The immune effector cell of claim 12 for use as a medicament.

14. The immune effector cell of claim 12 for use in treating a tumor in a patient in need thereof.

15. The immune effector cell for use of claim 14 wherein the tumor is a solid tumor.
